# EUROPÄISCHE PATENTSCHRIFT

(11) **EP 0 882 027 B1**
(45) Veröffentlichungstag und Bekanntmachung des Hinweises auf die Patenterteilung: **02.07.2003**
(21) Anmeldenummer: 97904411.2
(22) Anmeldetag: 10.02.1997
(51) Int. Cl.: C07D 253/06, A01N 43/707

(54) **SUBSTITUIERTE 2-ARYL-1,2,4-TRIAZIN-3,5-DI(THI)ONE ALS HERBIZIDE**
SUBSTITUTED 2-ARYL-1,2,4-TRIAZINE-3,5-DI(THI)ONES AS HERBICIDES
2-ARYL-1,2,4-TRIAZINE -3,5-DI(THI)ONES SUBSTITUEES UTILISEES COMME HERBICIDES

(30) Priorität: 22.02.1996 DE 19606594
(43) Veröffentlichungstag der Anmeldung: 09.12.1998
(73) Patentinhaber: Bayer CropScience AG, 40789 Monheim (DE)
(72) Erfinder: LINKER, Karl-Heinz, D-51377 Leverkusen (DE); FINDEISEN, Kurt, D-51375 Leverkusen (DE); DOLLINGER, Markus, D-51381 Leverkusen (DE); SANTEL, Hans-Joachim, D-51371 Leverkusen (DE)
(86) Internationale Anmeldenummer: EP9700610
(87) Internationale Veröffentlichungsnummer: WO97030980

(56) Entgegenhaltungen:
- EP-A- 0 011 693
- WO-A-86/00072
- US-A- 4 956 004

## Beschreibung

Die Erfindung betrifft neue substituierte 2-Aryl-1,2,4-triazin-3,5-di(thi)one, Verfahren zu ihrer Herstellung und ihre Verwendung als Herbizide.

Aus EP 11693, EP 271170, WO 86/00072, US 4755217, US 4878941, US 4956004, US 5262390, US 5344812 ist bekannt, dass bestimmte substituierte 2-Aryl-1,2,4-triazin-(thi)one herbizide Eigenschaften aufweisen. Die darin beschriebenen Verbindungen haben jedoch keine nennenswerte Bedeutung erlangt.

Es wurden nun die neuen substituierten 2-Aryl-1,2,4-triazin-3,5-di(thi)one der allgemeinen Formel (I) gefunden, 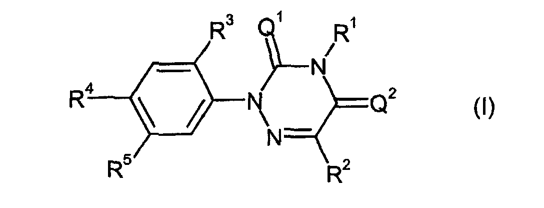 in welcher
- Q¹: für Sauerstoff oder Schwefel steht,
- Q²: für Sauerstoff oder Schwefel steht,
- R¹: für Wasserstoff, Cyano, Amino, für jeweils gegebenenfalls durch Cyano, Fluor, Chlor, C₁-C₄-Alkoxy oder C₁-C₄-Alkylthio substituiertes Alkyl, Alkoxy, Alkylamino, Dialkylamino, Alkylcarbonyl oder Alkoxycarbonyl mit jeweils 1 bis 6 Kohlenstoffatomen in den Alkylgruppen, für jeweils gegebenenfalls durch Fluor, Chlor oder Brom substituiertes Alkenyl, Alkenylcarbonyl, Alkenyloxycarbonyl, Alkinyl, Alkinylcarbonyl oder Alkinyloxycarbonyl mit jeweils 2 bis 6 Kohlenstoffatomen in den Alkenyl- bzw. Alkinyl-gruppen, oder für jeweils gegebenenfalls durch Cyano, Fluor, Chlor, Brom oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil steht,
- R²: für Wasserstoff, Halogen, Nitro, Carboxy, Cyano, Thiocarbamoyl, Amino, für jeweils gegebenenfalls durch Cyano, Fluor, Chlor, C₁-C₄-Alkoxy oder C₁-C₄-Alkylthio substituiertes Alkyl, Alkoxy, Alkylthio, Alkylamino oder Dialkylamino mit jeweils 1 bis 6 Kohlenstoffatomen in den Alkylgruppen, für jeweils gegebenenfalls durch Fluor, Chlor oder Brom substituiertes Alkenyl, Alkenyloxy, Alkenylthio, Alkinyl, Alkinyloxy oder Alkinylthio mit jeweils 2 bis 6 Kohlenstoffatomen in den Alkenyl- oder Alkinyl-gruppen, oder für jeweils gegebenenfalls durch Cyano, Fluor, Chlor, Brom oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 6 Kohlenstoffatomen im Alkylteil steht,
- R³: für Fluor, Chlor oder Brom steht,
- R⁴: für Cyano oder Thiocarbamoyl steht und
- R⁵: für die Gruppierung -A¹-A²-A³ steht,
in welcher
A¹ für Sauerstoff steht,
A² für eine Einfachbindung steht, und
A³ für jeweils gegebenenfalls durch Fluor oder Chlor substituiertes Alkenyl oder Alkinyl mit jeweils 2 bis 6 Kohlenstoffatomen in den Alkenyl- oder Alkinylgruppen steht, oder
A¹ für jeweils gegebenenfalls durch Fluor oder Chlor substituiertes Alkenyl oder Alkinyl mit jeweils 2 bis 6 Kohlenstoffatomen in den Alkenyl- oder Alkinylgruppen steht, oder
A¹ für die Gruppierung -N-A⁴- steht,
A² für eine Einfachbindung steht,
A³ für gegebenenfalls durch Fluor, Chlor oder C₁-C₄-Alkoxy substituiertes Alkylsulfonyl mit 1 bis 6 Kohlenstoffatomen in den Alkylgruppen steht, und
A⁴ für Wasserstoff oder C₁-C₄-Alkylsulfonyl steht.

Verbindungen der Formel (I), in denen R⁵ für F oder NH₂ steht, können als Ausgangsmaterialien für die Herstellung der Verbindungen der Formel (I) verwendet werden (= Verbindungen der Formel (I)').

Man erhält die neuen substituierten 2-Aryl-1,2,4-triazin-3,5-di(thi)one der allgemeinen Formel (I) oder (I'), wenn man
(a) 1,2,4-Triazin-3,5-di(thi)one der allgemeinen Formel (II), 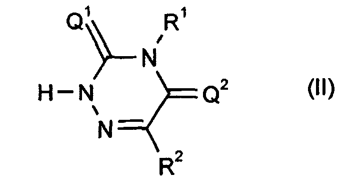 in welcher
   - Q¹, Q², R¹ und R²: die oben angegebenen Bedeutungen haben, 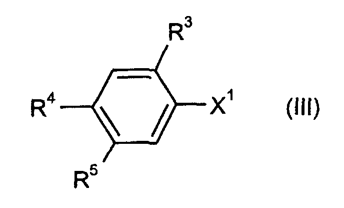
   in welcher
   - R³, R⁴ und R⁵: die oben angegebenen Bedeutungen haben und
   - X¹: für Halogen steht,
   gegebenenfalls in Gegenwart eines Verdünnungsmittels und gegebenenfalls in Gegenwart eines Reaktionshilfsmittels umsetzt, oder wenn man
(b) 2-Aryl-1,2,4-triazin-3,5-di(thi)one der allgemeinen Formel (Ia), 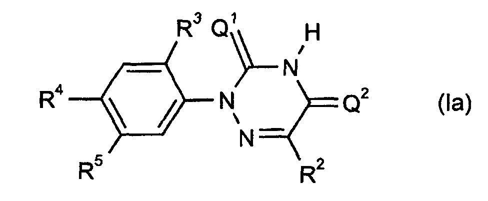 in welcher
   - Q¹, Q², R², R³, R⁴ und R⁵: die oben angegebenen Bedeutungen haben
   mit Alkylierungsmitteln der allgemeinen Formel (IV),

   R¹-X² (IV)

   in welcher
   - R¹: die oben angegebene Bedeutung hat und
   - X²: für Halogen oder die Gruppierung -O-SO₂-O-R¹ steht,
   gegebenenfalls in Gegenwart eines Verdünnungsmittels und gegebenenfalls in Gegenwart eines Reaktionshilfsmittels umsetzt,
   oder wenn man
(c) Arylhydrazone der allgemeinen Formel (V), 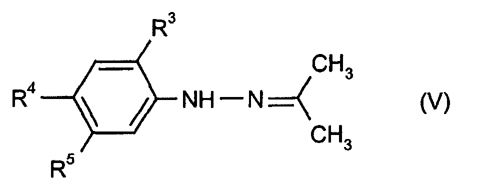 in welcher
   - R³, R⁴ und R⁵: die oben angegebenen Bedeutungen haben
   mit Alkalimetall(thio)cyanaten der allgemeinen Formel (VI),

   M-Q¹-CN (VI)

   in welcher
   - Q¹: die oben angegebene Bedeutung hat und
   - M: für ein Alkalimetallatom steht,
   gegebenenfalls in Gegenwart eines Verdünnungsmittels und gegebenenfalls in Gegenwart eines Reaktionshilfsmittels umsetzt und die hierbei gebildeten 2-Aryl-1,2,4-triazolidin-3-(thi)one der allgemeinen Formel (VII), 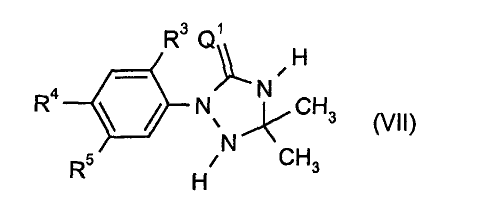 in welcher
   - Q^{1,} R³, R⁴ und R⁵: die oben angegebenen Bedeutungen haben
   mit Ketocarbonsäuren der allgemeinen Formel (VIII), 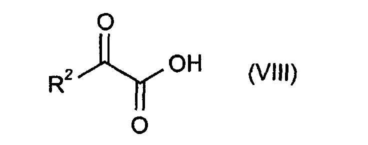 in welcher
   - R²: die oben angegebene Bedeutung hat
   gegebenenfalls in Gegenwart eines Verdünnungsmittels und gegebenenfalls in Gegenwart eines Reaktionshilfsmittels umsetzt,
   oder wenn man
(d) Aryl-amine der allgemeinen Formel (IX), 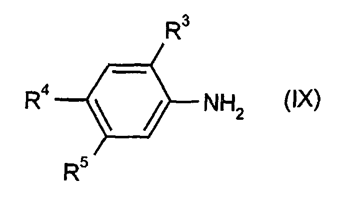 in welcher
   - R³, R⁴ und R⁵: die oben angegebenen Bedeutungen haben
   diazotiert und im Anschluss daran mit einem Cyanacetylcarbamidsäureester der allgemeinen Formel (X), 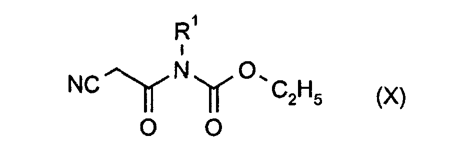 in welcher
   - R¹: die oben angegebene Bedeutung hat,
   gegebenenfalls in Gegenwart eines Verdünnungsmittels und gegebenenfalls in Gegenwart eines Reaktionshilfsmittels umsetzt,
   und die so erhaltenen Verbindungen der allgemeinen Formel (XI), 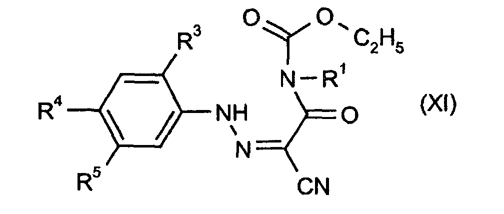 in welcher
   - R¹, R³, R⁴ und R⁵: die oben angegebenen Bedeutungen haben
   gegebenenfalls in Gegenwart eines Verdünnungsmittels und gegebenenfalls in Gegenwart eines Reaktionshilfsmittels cyclisiert,
   oder wenn man
(e) Arylhydrazine der allgemeinen Formel (XII) 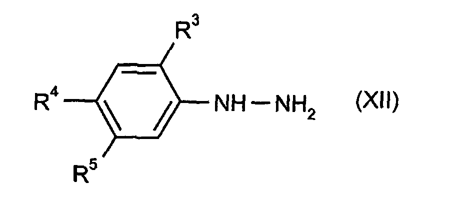 in welcher
   - R³, R⁴ und R⁵: die oben angegebenen Bedeutungen haben
   mit Ketocarbonsäure-Derivaten der allgemeinen Formel (XIII), 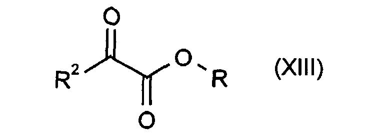 in welcher
   - R²: die oben angegebene Bedeutung hat und
   - R: für Wasserstoff oder Alkyl steht,
   gegebenenfalls in Gegenwart eines Verdünnungsmittels und gegebenenfalls in Gegenwart eines Reaktionshilfsmittels umsetzt und die so erhaltenen Verbindungen der allgemeinen Formel (XIV), 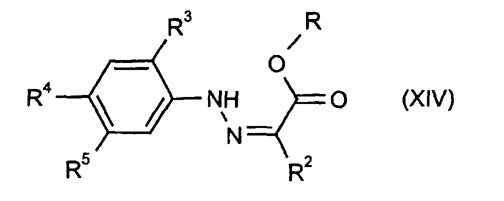 in welcher
   - R²,R³, R⁴ und R⁵: die oben angegebenen Bedeutungen haben und
   - R: für Wasserstoff oder Alkyl steht,
   gegebenenfalls in Gegenwart eines Verdünnungsmittels und gegebenenfalls in Gegenwart eines Reaktionshilfsmittels mit Iso(thio)cyanaten der allgemeinen Formel (XV),

   R¹-N=C=Q¹ (XV)

   in welcher
   - Q¹ und R¹: die oben angegebenen Bedeutungen haben,
   gegebenenfalls in Gegenwart eines Verdünnungsmittels und gegebenenfalls in Gegenwart eines Reaktionshilfsmittels umsetzt
   und die so erhaltenen Verbindungen der allgemeinen Formel (XVI), 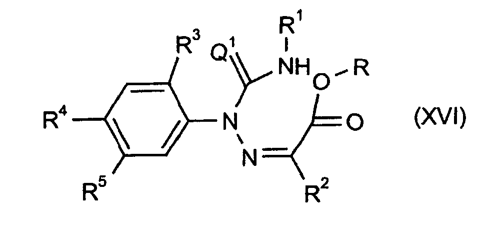 in welcher
   - Q¹, R¹, R², R³, R⁴ und R⁵: die oben angegebenen Bedeutungen haben und
   - R: für Wasserstoff oder Alkyl steht,
   gegebenenfalls in Gegenwart eines Verdünnungsmittels und gegebenenfalls in Gegenwart eines Reaktionshilfsmittels cyclisiert,
   oder man erhält
(f) 2-Aryl-1,2,4-triazin-3,5-dion-6-carbonsäuren der allgemeinen Formel (XIX), 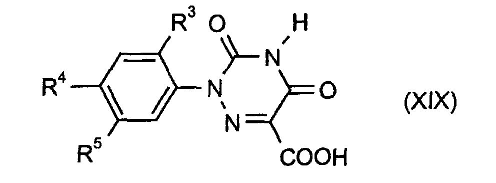 in welcher
   - R³, R⁴ und R⁵: die oben angegebenen Bedeutung haben,
   wenn man Arylamine der allgemeinen Formel (IX), 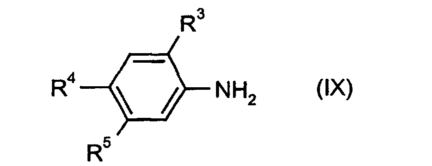 in welcher
   - R³, R⁴ und R⁵: die oben angegebenen Bedeutungen haben
   diazotiert und im Anschluss daran mit dem Malonyldiurethan der Formel (XVII),

   CH₂(CO-NH-COOC₂H₅)₂ (XVII)

   gegebenenfalls in Gegenwart eines Verdünnungsmittels und gegebenenfalls in Gegenwart eines Reaktionshilfsmittels umsetzt und anschließend cyclisiert.

Verbindungen der allgemeinen Formel (I), bei denen R⁵ für Fluor oder NH₂ steht, können auch nach üblichen Methoden in Verbindungen der allgemeinen Formel (I) gemäß obiger Definition umgewandelt werden, beispielsweise durch nucleophile Substitution (z.B. R⁵: F → NH₂, OCH₂CH=CH₂, NHSO₂CH₃); oder durch weitere Umwandlungen funktioneller Gruppen (z.B. R²: COOH → H, COOH → COCl, COOCH₃, COCI →CONH₂, CN → CSNH₂; R⁴: CN → CSNH₂; NH₂ → F, Cl, Br, NHSO₂CH₃) oder durch Oxidation oder Schwefelung (z.B. Q¹, Q²: O → S, oder S → O), vergl. auch die Herstellungsbeispiele.

Die neuen substituierten 2-Aryl-1,2,4-triazin-3,5-di(thi)one der allgemeinen Formel (I) zeichnen sich durch starke und selektive herbizide Wirksamkeit aus.

In den Definitionen sind die gesättigten oder ungesättigten Kohlenwasserstoffketten, wie Alkyl, Alkenyl oder Alkinyl jeweils geradkettig oder verzweigt.

Gegenstand der Erfindung sind vorzugsweise Verbindungen der Formel (I), in welcher
- R¹: für Wasserstoff, Cyano, Amino, für jeweils gegebenenfalls durch Cyano, Fluor, Chlor, Methoxy oder Ethoxy substituiertes Methyl, Ethyl, n- oder i-Propyl, n-, i- s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino, Dimethylamino, Diethylamino, Acetyl, Propionyl, n- oder i-Butyroyl, Methoxycarbonyl, Ethoxycarbonyl, n- oder i-Propoxycarbonyl, für jeweils gegebenenfalls durch Fluor, Chlor oder Brom substituiertes Propenyl, Butenyl, Propenylcarbonyl, Butenylcarbonyl, Propenyloxycarbonyl, Butenyloxycarbonyl, für Propinyl, Butinyl, Propinylcarbonyl, Butinylcarbonyl, Propinyloxycarbonyl oder Butinyloxycarbonyl, oder für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
- R²: für Wasserstoff, Halogen, Nitro, Carboxy, Cyano, Thiocarbamoyl, Amino, für jeweils gegebenenfalls durch Cyano, Fluor, Chlor, Methoxy oder Ethoxy substituiertes Methyl, Ethyl, n- oder i-Propyl, n-, i- s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino, für jeweils gegebenenfalls durch Fluor, Chlor oder Brom substituiertes Propenyl, Propenyloxy, Propenylthio, Butenyl, Butenyloxy oder Butenylthio, für Propinyl, Propinyloxy, Propinylthio, Butinyl, Butinyloxy oder Butinylthio, oder für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
- R³: für Fluor oder Chlor steht,
- R⁴: für Cyano oder Thiocarbamoyl steht und
- R⁵: für die Gruppierung -A¹-A²-A³ steht,
in welcher
A¹ für Sauerstoff steht,
A² für eine Einfachbindung steht,
A³ weiterhin für jeweils gegebenenfalls durch Fluor oder Chlor substituiertes Propenyl, Butenyl, Propinyl oder Butinyl steht, oder
A¹ für die Gruppierung -NH-,
A² für eine Einfachbindung, und
A³ für jeweils gegebenenfalls durch Fluor, Chlor, Methoxy oder Ethoxy substituiertes Methylsulfonyl, Ethylsulfonyl, n- oder i-Propylsulfonyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Beispiele für die erfindungsgemäßen Verbindungen der Formel (I) und der Formel (I') sind in den nachstehenden Gruppen aufgeführt.

### Gruppe 1

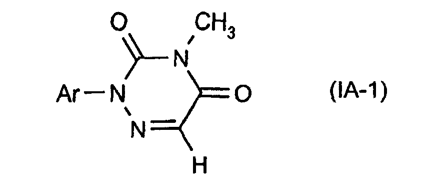

- Ar: hat hierbei beispielhaft die im Folgenden aufgeführten Bedeutungen:

2-Chlor-5-fluor-4-cyano-phenyl, 2,5-Difluor-4-cyano-phenyl, 2,5-Difluor-4-thiocarbamoyl-phenyl, 2-Chlor-4-cyano-5-(2-propinyloxy)-phenyl, 2-Fluor-4-cyano-5-(1-methyl-2-propinyloxy)-phenyl, 2-Fluor-4-cyano-5-(2-propenyloxy)-phenyl, 2-Chlor-4-cyano-5-methylsulfonylamino-phenyl, 2-Fluor-4-cyano-5-ethylsulfonylamino-phenyl, 2-Fluor-4-thiocarbamoyl-5-methylsulfonyl-phenyl, 2-Chlor-4-cyano-5-ethylsulfonylamino-phenyl, 2-Fluor-4-cyano-5-i-propylsulfonylamino-phenyl, 2-Chlor-4-thiocarbamoyl-5-ethylsulfonylamino-phenyl, 2-Fluor-4-cyano-5-trifluormethylsulfonylamino-phenyl, 2-Fluor-4-cyano-5-(2,2-difluorethylsulfonylamino)-phenyl, 2-Fluor-4-cyano-5-t-butylsulfonylamino-phenyl, 2-Fluor-4-cyano-5-(bis-ethylsulfonylamino)-phenyl, 2-Fluor-4-cyano-5-(N-methylsulfonyl-ethylsulfonylamino)-phenyl, 2-Fluor-4-cyano-5-amino-phenyl, 2-Fluor-4-cyano-5-(2-chlor-2-propenyloxy)-phenyl.

### Gruppe 2

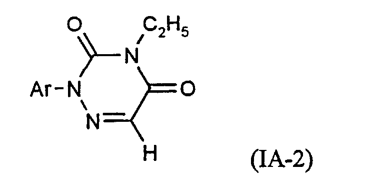

Ar hat hierbei die oben in Gruppe 1 aufgeführten Bedeutungen.

### Gruppe 3

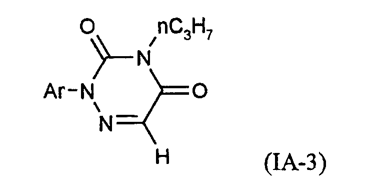

Ar hat hierbei die oben in Gruppe 1 aufgeführten Bedeutungen.

### Gruppe 4

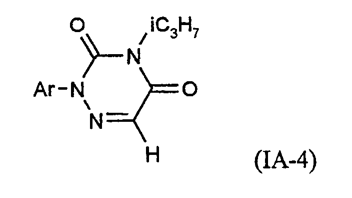

Ar hat hierbei die oben in Gruppe 1 aufgeführten Bedeutungen.

### Gruppe 5

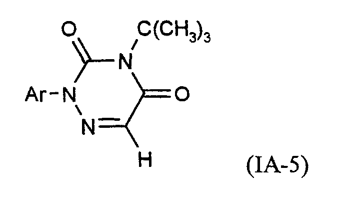

Ar hat hierbei die oben in Gruppe 1 aufgeführten Bedeutungen.

### Gruppe 6

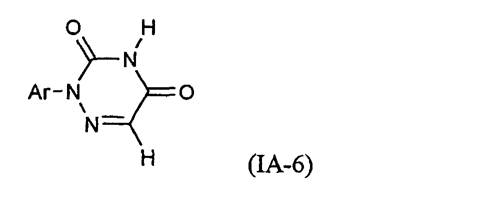

Ar hat hierbei die oben in Gruppe 1 aufgeführten Bedeutungen.

### Gruppe 7

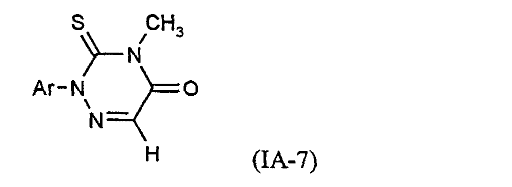

Ar hat hierbei die oben in Gruppe 1 aufgeführten Bedeutungen.

### Gruppe 8

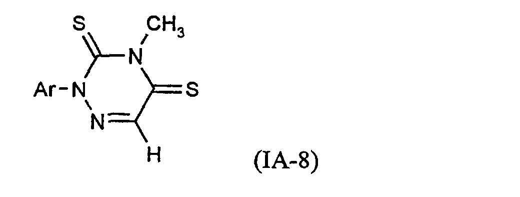

Ar hat hierbei die oben in Gruppe 1 aufgeführten Bedeutungen.

### Gruppe 9

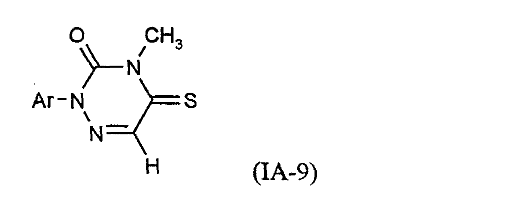

Ar hat hierbei die oben in Gruppe 1 aufgeführten Bedeutungen.

### Gruppe 10

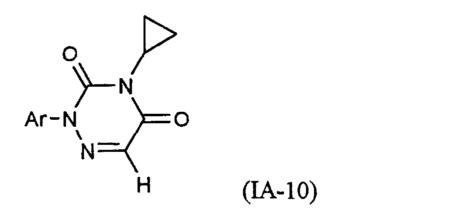

Ar hat hierbei die oben in Gruppe 1 aufgeführten Bedeutungen.

### Gruppe 11

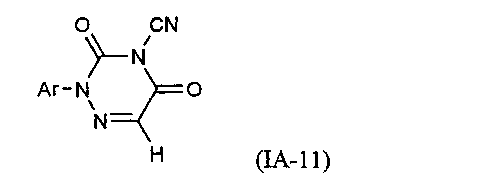

Ar hat hierbei die oben in Gruppe 1 aufgeführten Bedeutungen.

### Gruppe 12

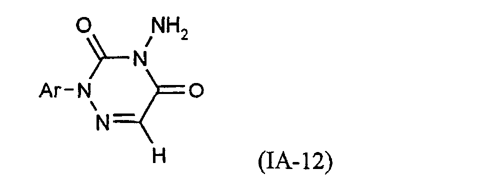

Ar hat hierbei die oben in Gruppe 1 aufgeführten Bedeutungen.

### Gruppe 13

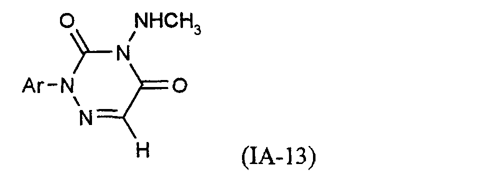

Ar hat hierbei die oben in Gruppe 1 aufgeführten Bedeutungen.

### Gruppe 14

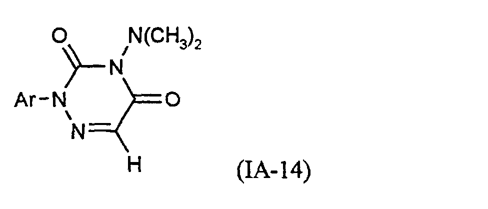

Ar hat hierbei die oben in Gruppe 1 aufgeführten Bedeutungen.

### Gruppe 15

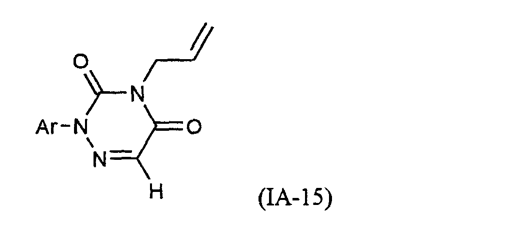

Ar hat hierbei die oben in Gruppe 1 aufgeführten Bedeutungen.

### Gruppe 16

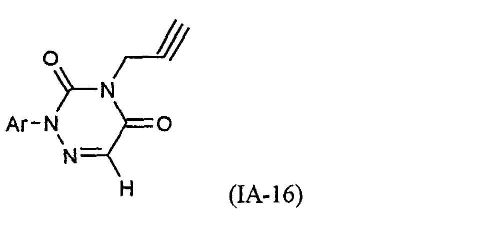

Ar hat hierbei die oben in Gruppe 1 aufgeführten Bedeutungen.

### Gruppe 17

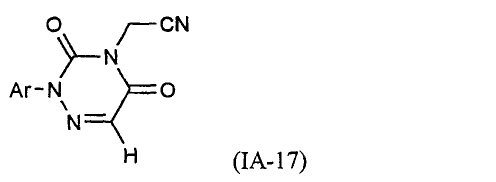

Ar hat hierbei die oben in Gruppe 1 aufgeführten Bedeutungen.

### Gruppe 18

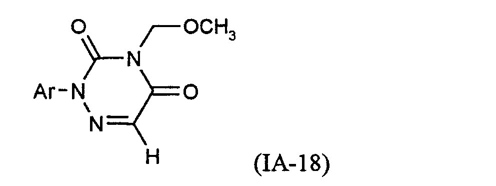

Ar hat hierbei die oben in Gruppe 1 aufgeführten Bedeutungen.

### Gruppe 19

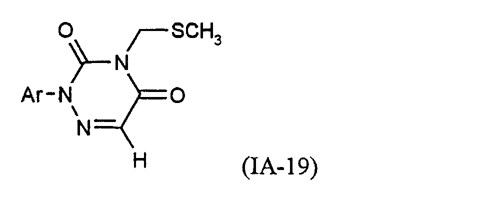

Ar hat hierbei die oben in Gruppe 1 aufgeführten Bedeutungen.

### Gruppe 20

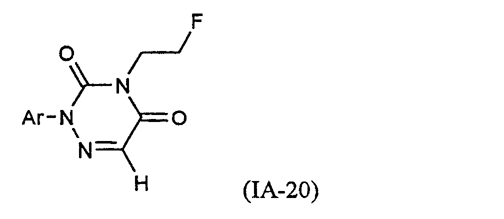

Ar hat hierbei die oben in Gruppe 1 aufgeführten Bedeutungen.

### Gruppe 21

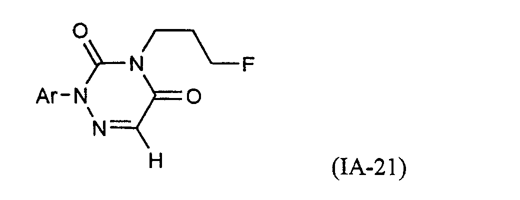

Ar hat hierbei die oben in Gruppe 1 aufgeführten Bedeutungen.

### Gruppe 22

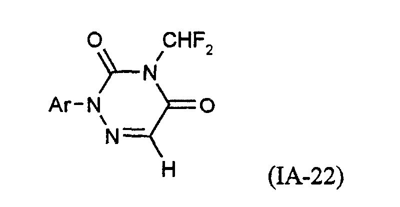

Ar hat hierbei die oben in Gruppe 1 aufgeführten Bedeutungen.

### Gruppe 23

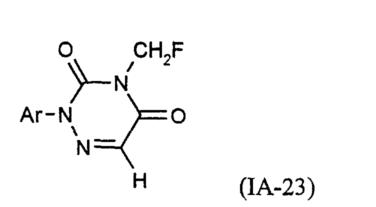

Ar hat hierbei die oben in Gruppe 1 aufgeführten Bedeutungen.

### Gruppe 24

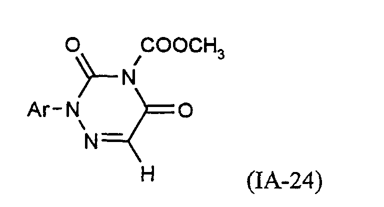

Ar hat hierbei die oben in Gruppe 1 aufgeführten Bedeutungen.

### Gruppe 25

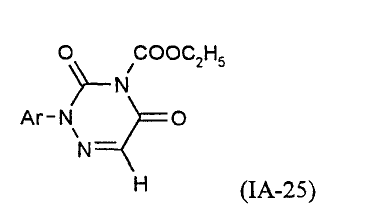

Ar hat hierbei die oben in Gruppe 1 aufgeführten Bedeutungen.

### Gruppe 26

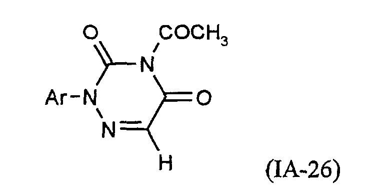

Ar hat hierbei die oben in Gruppe 1 aufgeführten Bedeutungen.

### Gruppe 27

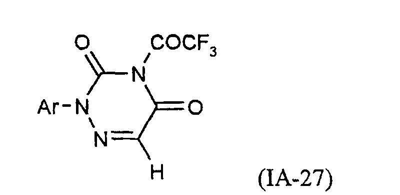

Ar hat hierbei die oben in Gruppe 1 aufgeführten Bedeutungen.

### Gruppe 28

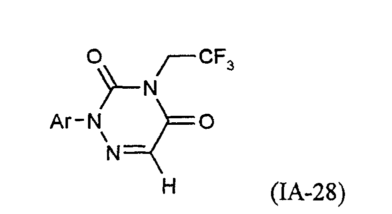

Ar hat hierbei die oben in Gruppe 1 aufgeführten Bedeutungen.

### Gruppe 29

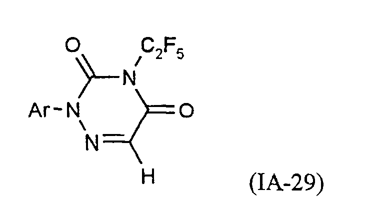

Ar hat hierbei die oben in Gruppe 1 aufgeführten Bedeutungen

### Gruppe 30

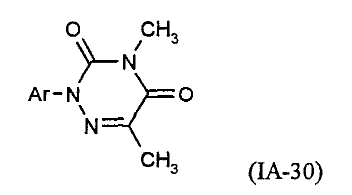

Ar hat hierbei die oben in Gruppe 1 aufgeführten Bedeutungen.

### Gruppe 31

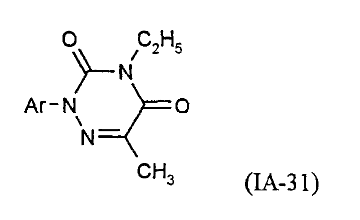

Ar hat hierbei die oben in Gruppe 1 aufgeführten Bedeutungen.

### Gruppe 32

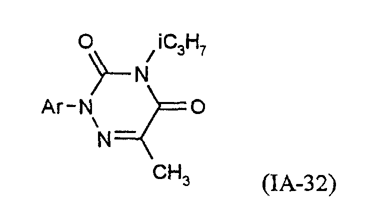

Ar hat hierbei die oben in Gruppe 1 aufgeführten Bedeutungen.

### Gruppe 33

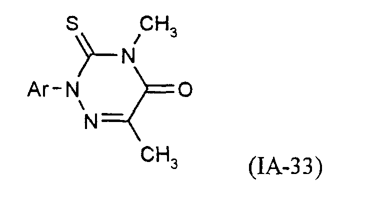

Ar hat hierbei die oben in Gruppe 1 aufgeführten Bedeutungen.

### Gruppe 34

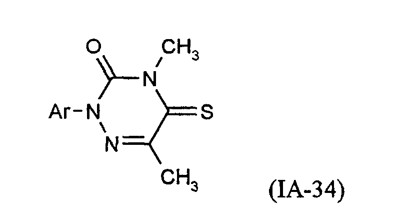

Ar hat hierbei die oben in Gruppe 1 aufgeführten Bedeutungen.

### Gruppe 35

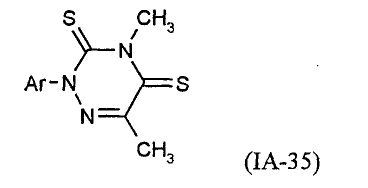

Ar hat hierbei die oben in Gruppe 1 aufgeführten Bedeutungen.

### Gruppe 36

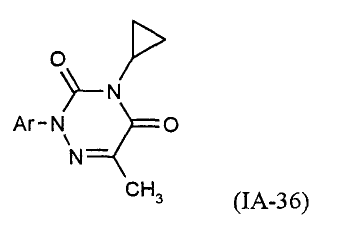

Ar hat hierbei die oben in Gruppe 1 aufgeführten Bedeutungen.

### Gruppe 37

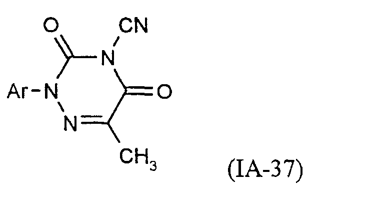

Ar hat hierbei die oben in Gruppe 1 aufgeführten Bedeutungen.

### Gruppe 38

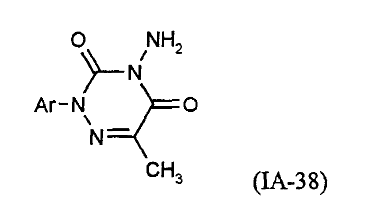

Ar hat hierbei die oben in Gruppe 1 aufgeführten Bedeutungen.

### Gruppe 39

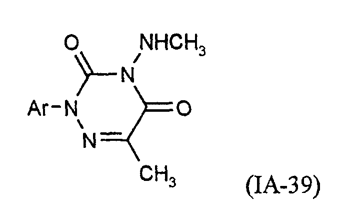

Ar hat hierbei die oben in Gruppe 1 aufgeführten Bedeutungen.

### Gruppe 40

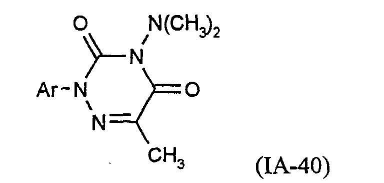

Ar hat hierbei die oben in Gruppe 1 aufgeführten Bedeutungen.

### Gruppe 41

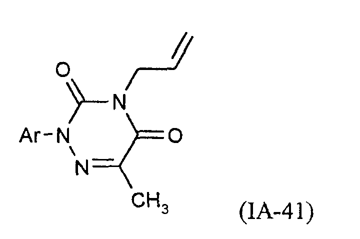

Ar hat hierbei die oben in Gruppe 1 aufgeführten Bedeutungen.

### Gruppe 42

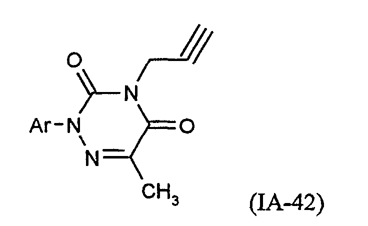

Ar hat hierbei die oben in Gruppe 1 aufgeführten Bedeutungen.

### Gruppe 43

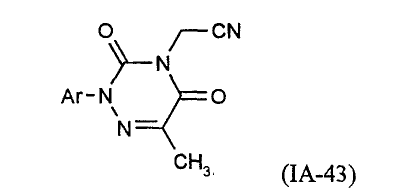

Ar hat hierbei die oben in Gruppe 1 aufgeführten Bedeutungen.

### Gruppe 44

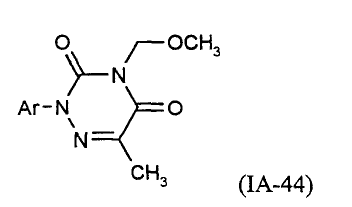

Ar hat hierbei die oben in Gruppe 1 aufgeführten Bedeutungen.

### Gruppe 45

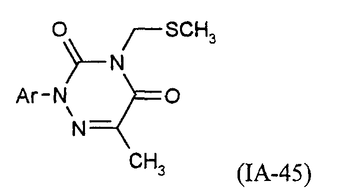

Ar hat hierbei die oben in Gruppe 1 aufgeführten Bedeutungen.

### Gruppe 46

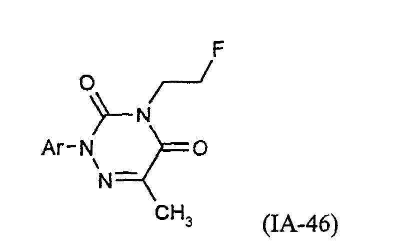

Ar hat hierbei die oben in Gruppe 1 aufgeführten Bedeutungen.

### Gruppe 47

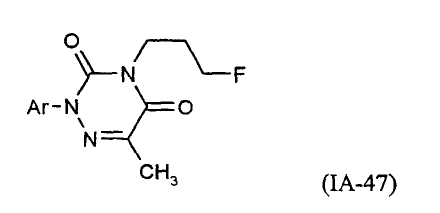

Ar hat hierbei die oben in Gruppe 1 aufgeführten Bedeutungen.

### Gruppe 48

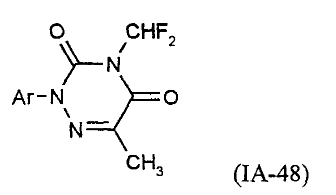

Ar hat hierbei die oben in Gruppe 1 aufgeführten Bedeutungen.

### Gruppe 49

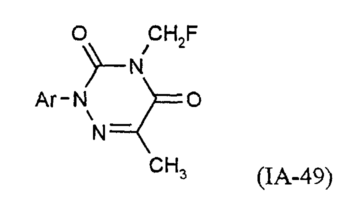

Ar hat hierbei die oben in Gruppe 1 aufgeführten Bedeutungen.

### Gruppe 50

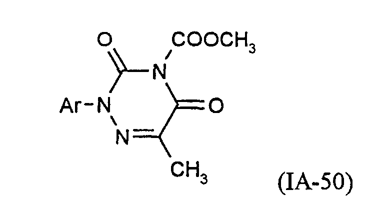

Ar hat hierbei die oben in Gruppe 1 aufgeführten Bedeutungen.

### Gruppe 51

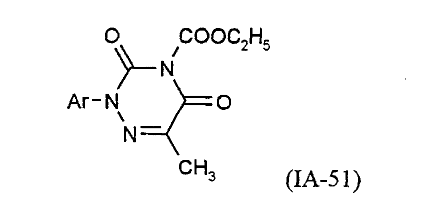

Ar hat hierbei die oben in Gruppe 1 aufgeführten Bedeutungen.

### Gruppe 52

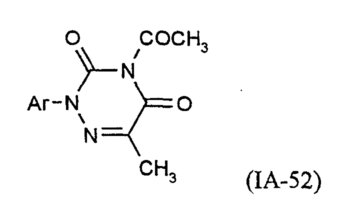

Ar hat hierbei die oben in Gruppe 1 aufgeführten Bedeutungen.

### Gruppe 53

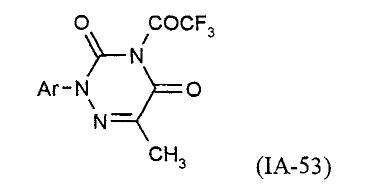

Ar hat hierbei die oben in Gruppe 1 aufgeführten Bedeutungen.

### Gruppe 54

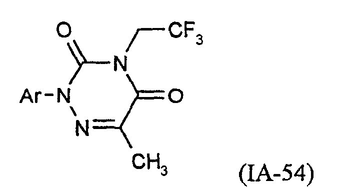

Ar hat hierbei die oben in Gruppe 1 aufgeführten Bedeutungen.

### Gruppe 55

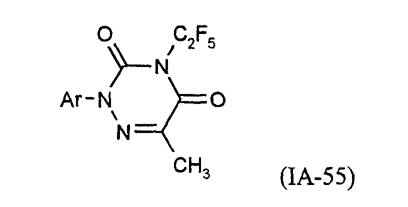

Ar hat hierbei die oben in Gruppe 1 aufgeführten Bedeutungen.

### Gruppe 56

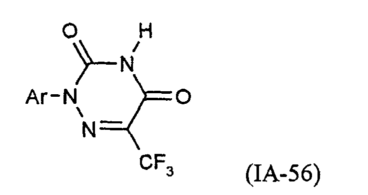

Ar hat hierbei die oben in Gruppe 1 aufgeführten Bedeutungen.

### Gruppe 57

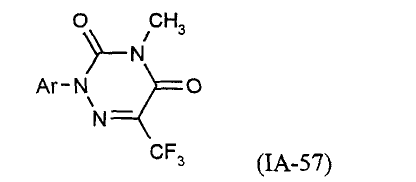

Ar hat hierbei die oben in Gruppe 1 aufgeführten Bedeutungen.

### Gruppe 58

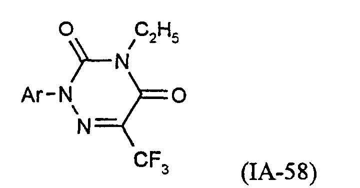

Ar hat hierbei die oben in Gruppe 1 aufgeführten Bedeutungen.

### Gruppe 59

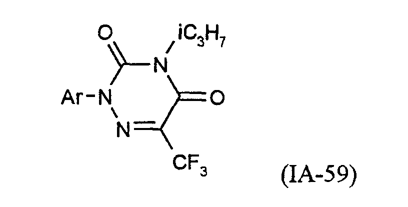

Ar hat hierbei die oben in Gruppe 1 aufgeführten Bedeutungen.

### Gruppe 60

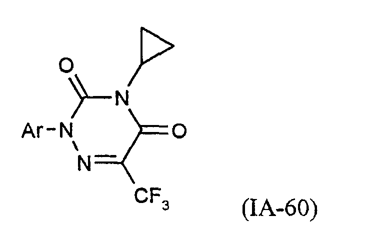

Ar hat hierbei die oben in Gruppe 1 aufgeführten Bedeutungen.

### Gruppe 61

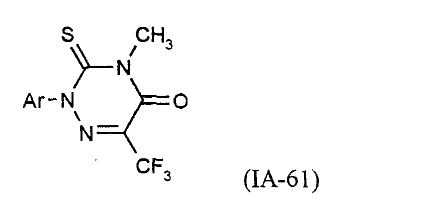

Ar hat hierbei die oben in Gruppe 1 aufgeführten Bedeutungen.

### Gruppe 62

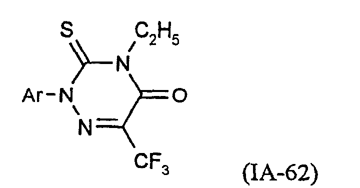

Ar hat hierbei die oben in Gruppe 1 aufgeführten Bedeutungen.

### Gruppe 63

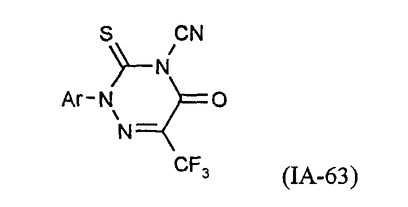

Ar hat hierbei die oben in Gruppe 1 aufgeführten Bedeutungen.

### Gruppe 64

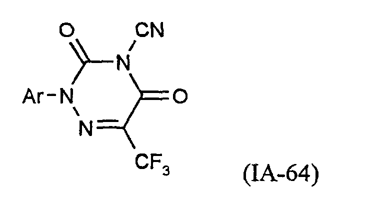

Ar hat hierbei die oben in Gruppe 1 aufgeführten Bedeutungen.

### Gruppe 65

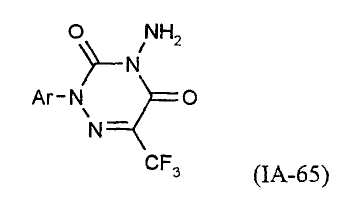

Ar hat hierbei die oben in Gruppe 1 aufgeführten Bedeutungen.

### Gruppe 67

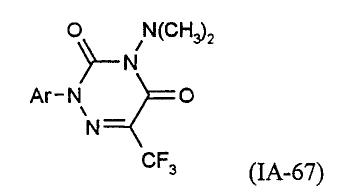

Ar hat hierbei die oben in Gruppe 1 aufgeführten Bedeutungen.

### Gruppe 68

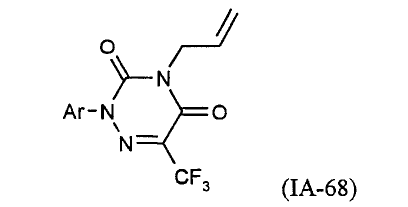

Ar hat hierbei die oben in Gruppe 1 aufgeführten Bedeutungen.

### Gruppe 69

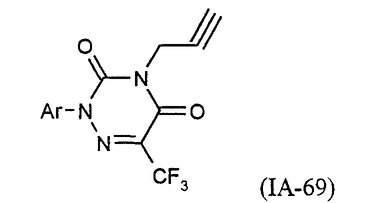

Ar hat hierbei die oben in Gruppe 1 aufgeführten Bedeutungen.

### Gruppe 70

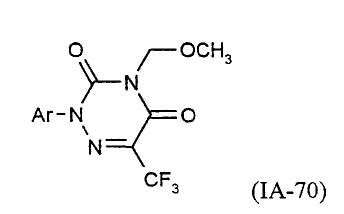

Ar hat hierbei die oben in Gruppe 1 aufgeführten Bedeutungen.

### Gruppe 71

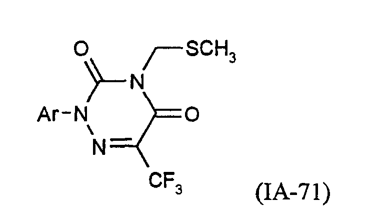

Ar hat hierbei die oben in Gruppe 1 aufgeführten Bedeutungen.

### Gruppe 72

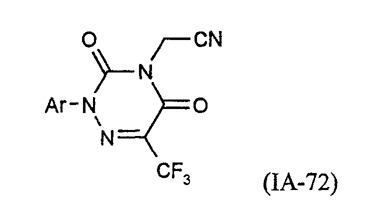

Ar hat hierbei die oben in Gruppe 1 aufgeführten Bedeutungen.

### Gruppe 73

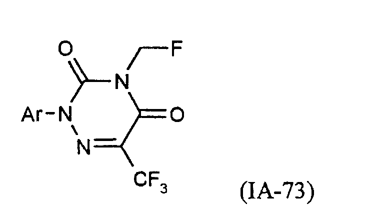

Ar hat hierbei die oben in Gruppe 1 aufgeführten Bedeutungen.

### Gruppe 74

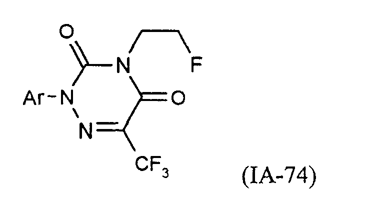

Ar hat hierbei die oben in Gruppe 1 aufgeführten Bedeutungen

### Gruppe 75

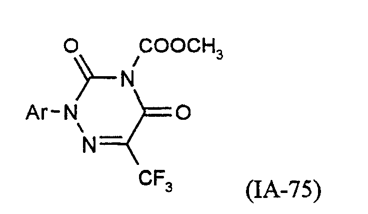

Ar hat hierbei die oben in Gruppe 1 aufgeführten Bedeutungen.

### Gruppe 76

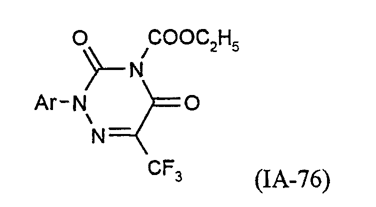

Ar hat hierbei die oben in Gruppe 1 aufgeführten Bedeutungen.

### Gruppe 77

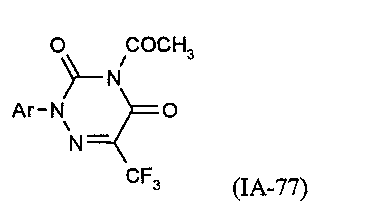

Ar hat hierbei die oben in Gruppe 1 aufgeführten Bedeutungen.

### Gruppe 78

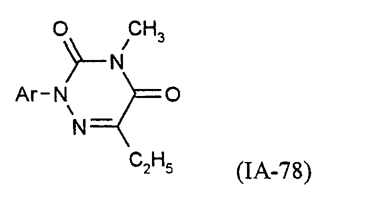

Ar hat hierbei die oben in Gruppe 1 aufgeführten Bedeutungen.

### Gruppe 79

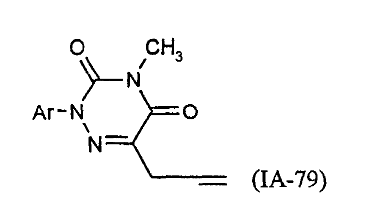

Ar hat hierbei die oben in Gruppe 1 aufgeführten Bedeutungen.

### Gruppe 80

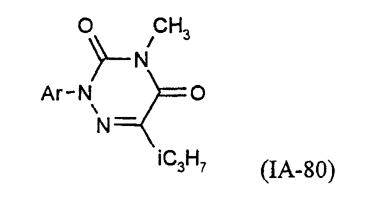

Ar hat hierbei die oben in Gruppe 1 aufgeführten Bedeutungen.

### Gruppe 81

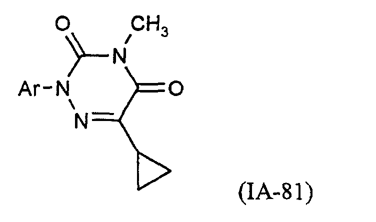

Ar hat hierbei die oben in Gruppe 1 aufgeführten Bedeutungen.

### Gruppe 82

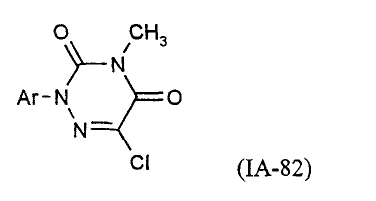

Ar hat hierbei die oben in Gruppe 1 aufgeführten Bedeutungen.

### Gruppe 83

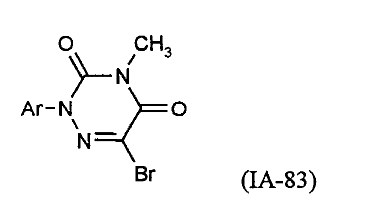

Ar hat hierbei die oben in Gruppe 1 aufgeführten Bedeutungen.

### Gruppe 84

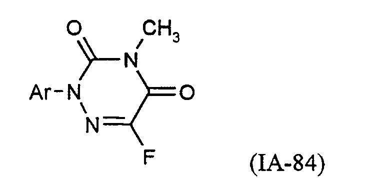

Ar hat hierbei die oben in Gruppe 1 aufgeführten Bedeutungen.

### Gruppe 85

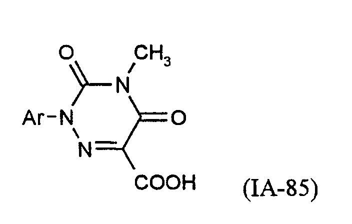

Ar hat hierbei die oben in Gruppe 1 aufgeführten Bedeutungen.

### Gruppe 86

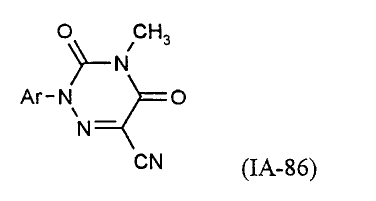

Ar hat hierbei die oben in Gruppe 1 aufgeführten Bedeutungen

### Gruppe 87

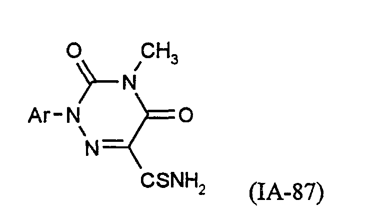

Ar hat hierbei die oben in Gruppe 1 aufgeführten Bedeutungen.

### Gruppe 88

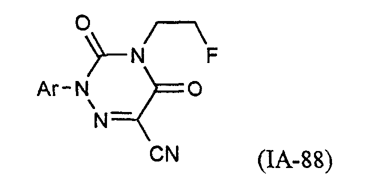

Ar hat hierbei die oben in Gruppe 1 aufgeführten Bedeutungen.

### Gruppe 89

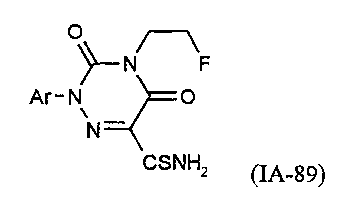

Ar hat hierbei die oben in Gruppe 1 aufgeführten Bedeutungen.

### Gruppe 90

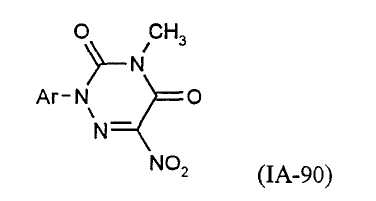

Ar hat hierbei die oben in Gruppe 1 aufgeführten Bedeutungen.

### Grupne 91

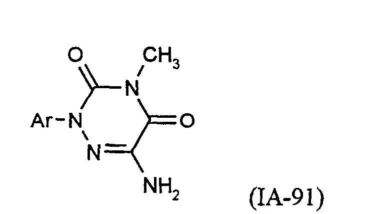

Ar hat hierbei die oben in Gruppe 1 aufgeführten Bedeutungen.

### Gruppe 92

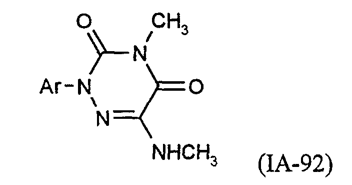

Ar hat hierbei die oben in Gruppe 1 aufgeführten Bedeutungen.

### Gruppe 93

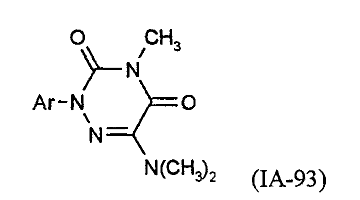

Ar hat hierbei die oben in Gruppe 1 aufgeführten Bedeutungen.

### Gruppe 94

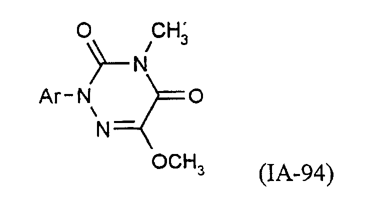

Ar hat hierbei die oben in Gruppe 1 aufgeführten Bedeutungen.

### Gruppe 95

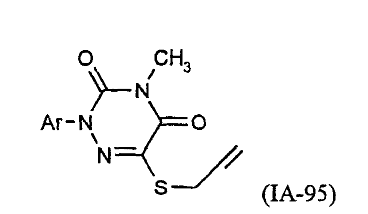

Ar hat hierbei die oben in Gruppe 1 aufgeführten Bedeutungen.

### Gruppe 96

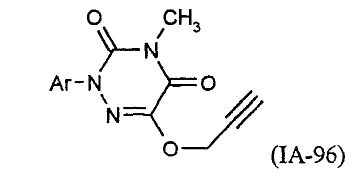

Ar hat hierbei die oben in Gruppe 1 aufgeführten Bedeutungen.

### Gruppe 97

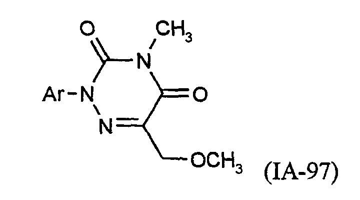

Ar hat hierbei die oben in Gruppe 1 aufgeführten Bedeutungen.

### Gruppe 98

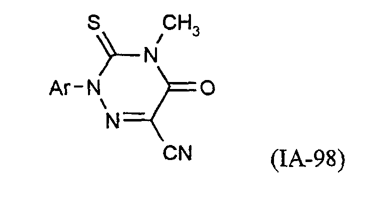

Ar hat hierbei die oben in Gruppe 1 aufgeführten Bedeutungen.

Verwendet man beispielsweise 4,6-Diethyl-1,2,4-triazin-3,5(2H,4H)-dion und 2,4,5-Trifluor-benzonitril als Ausgangsstoffe, so kann der Reaktionsablauf beim erfindungsgemäßen Verfahren (a) durch das folgende Formelschema skizziert werden:

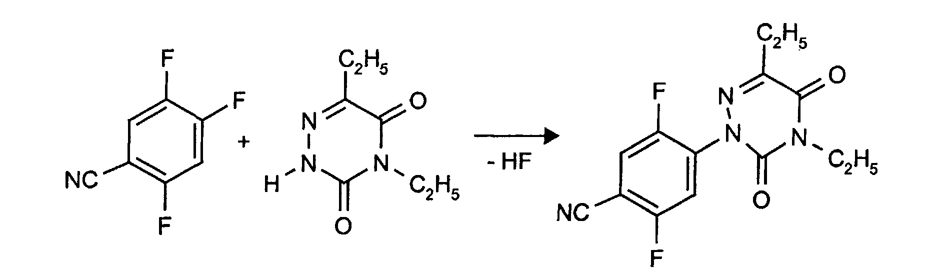

Verwendet man beispielsweise 2-(4-Cyano-5-ethoxy-2-fluor-phenyl)-6-trifluormethyl-1,2,4-triazin-3,5(2H,4H)-dion und Ethylbromid als Ausgangsstoffe, so kann der Reaktionsablauf beim erfindungsgemäßen Verfahren (b) durch das folgende Formelschema skizziert werden:

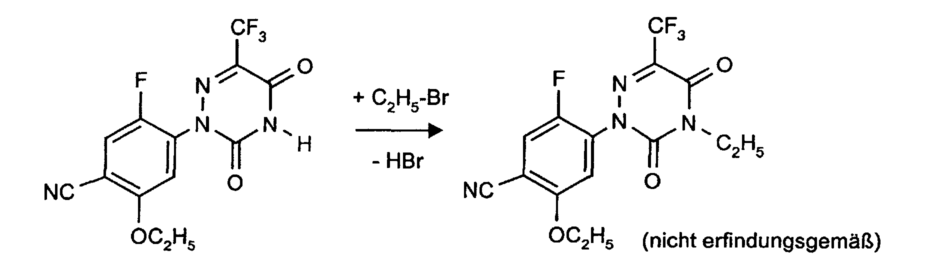

Verwendet man beispielsweise N-(5-Chlor-4-cyano-2-fluor-phenyl)-acetonhydrazon und Kaliumthiocyanat sowie in der Folgestufe Brenztraubensäure als Ausgangsstoffe, so kann der Reaktionsablauf beim erfindungsgemäßen Verfahren (c) durch das folgende Formelschema skizziert werden:

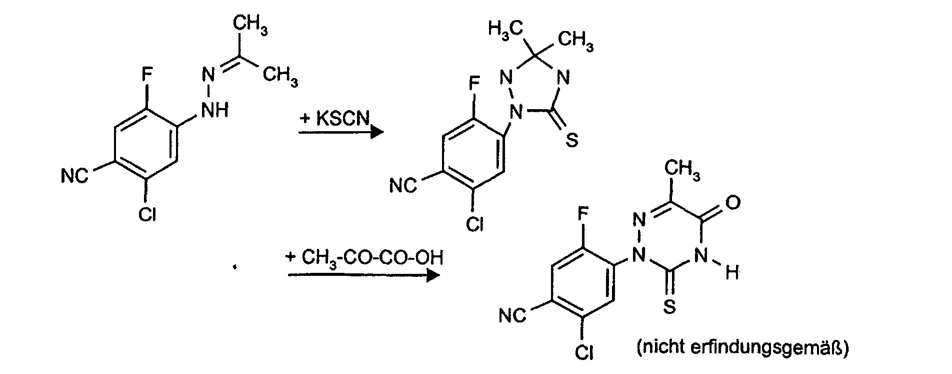

Verwendet man beispielsweise 4-Amino-3-fluor-2-methylthio-benzonitril und N-Cyanacetyl-O-ethyl-carbamidsäureester als Ausgangsstoffe so kann der Reaktionsablauf beim erfindungsgemäßen Verfahren (d) durch das folgende Formelschema skizziert werden:

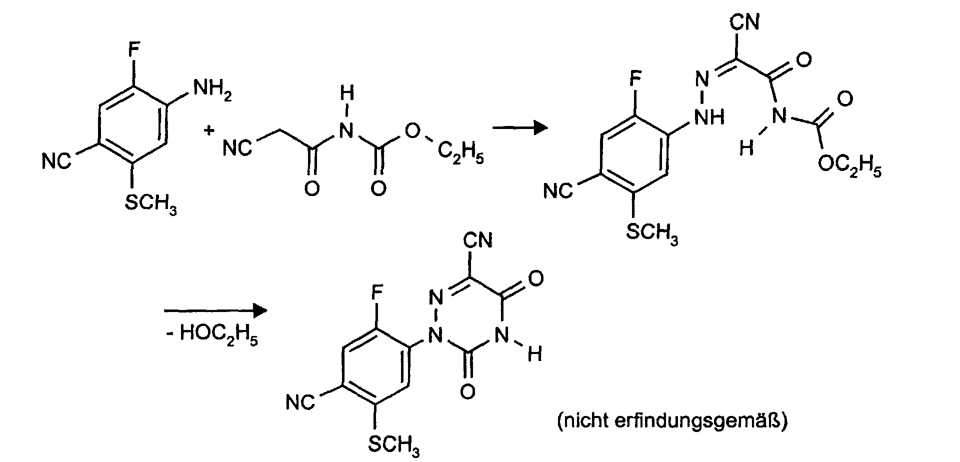

Verwendet man beispielsweise 2-Chlor-4-cyano-5-methyl-phenylhydrazin und Brenztraubensäure-methyl-ester sowie in der Folgestufe Ethylisothiocyanat als Ausgangsstoffe, so kann der Reaktionsablauf beim erfindungsgemäßen Verfahren (e) durch das folgende Formelschema skizziert werden:

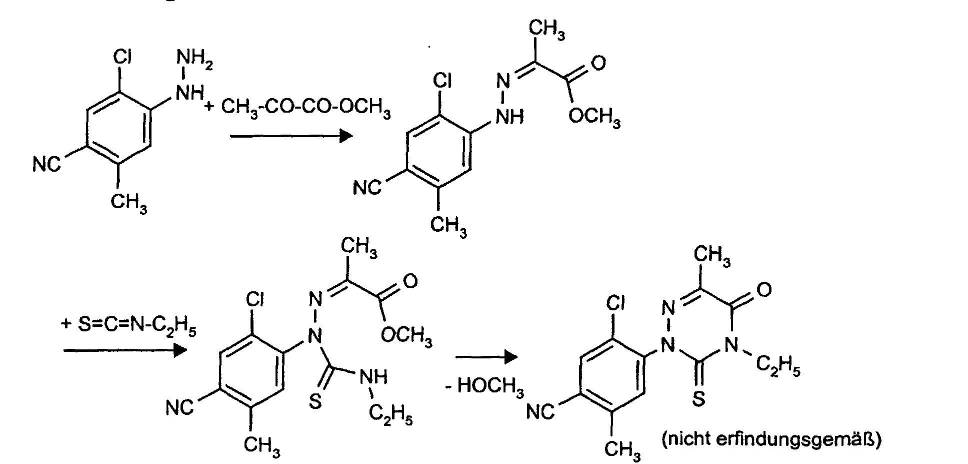

Verwendet man beispielsweise 4-Amino-2,5-dichlor-benzonitril und Natriumnitrit/Salzsäure sowie in der Folgestufe Malonyldiethylurethan als Ausgangsstoffe, so kann der Reaktionsablauf beim erfindungsgemäßen Verfahren (f) durch das folgende Formelschema skizziert werden:

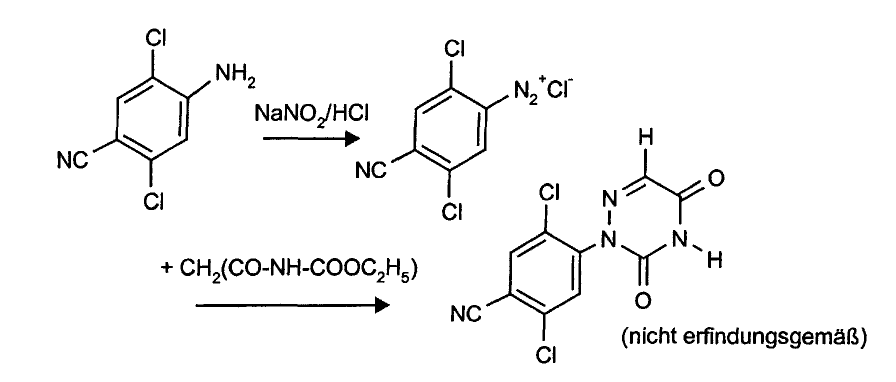

Die beim erfindungsgemäßen Verfahren (a) zur Herstellung von Verbindungen der Formel (I) als Ausgangsstoffe zu verwendenden 1,2,4-Triazin-3,5-di(thi)one sind durch die Formel (II) allgemein definiert. In der Formel (II) haben Q¹, Q², R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q¹, Q², R¹ und R² angegeben wurden.

Die Ausgangsstoffe der Formel (II) sind bekannt und/oder können nach bekannten Verfahren hergestellt werden (vgl. J. Am. Chem. Soc. 80 (1958), 976; J. Heterocycl. Chem. 16 (1979), 1649; J. Org. Chem. 23 (1958), 1951-1953).

Die beim erfindungsgemäßen Verfahren (a) weiter als Ausgangsstoffe zu verwendenden Halogenbenzol-Derivate sind durch die Formel (III) allgemein definiert. In der Formel (III) haben R³, R⁴ und R⁵ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R³, R⁴ und R⁵ angegeben wurden; X¹ steht vorzugsweise für Fluor, Chlor oder Brom, insbesondere für Fluor.

Die Ausgangsstoffe der Formel (III) sind bekannt und/oder können nach an sich bekannten Verfahren hergestellt werden (vgl. EP 191181, EP 370332, EP 431373, EP 441004).

Die beim erfindungsgemäßen Verfahren (b) als Ausgangsstoffe zu verwendenden 2-Aryl-1,2,4-triazin-3,5-di(thi)one sind durch die Formel (Ia) allgemein definiert. In der Formel (Ia) haben Q¹, Q², R² , R³, R⁴ und R⁵ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q¹, Q², R² , R³, R⁴ und R⁵ angegeben wurden.

Die Ausgangsstoffe der Formel (Ia) sind erfindungsgemäße, neue Verbindungen; sie können nach den erfindungsgemäßen Verfahren (a) sowie (c) bis (f) hergestellt werden.

Die beim erfindungsgemäßen Verfahren (b) weiter als Ausgangsstoffe zu verwendenden Alkylierungsmittel sind durch die Formel (IV) allgemein definiert. In der Formel (IV) hat R¹ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R¹ angegeben wurde; X² steht vorzugsweise für Fluor, Chlor, Brom, Iod, Methoxysulfonyloxy oder Ethoxysulfonyloxy, insbesondere für Chlor oder Brom.

Die Ausgangsstoffe der Formel (IV) sind bekannte organische Synthesechemikalien.

Die beim erfindungsgemäßen Verfahren (c) zur Herstellung von Verbindungen der Formel (I) als Ausgangsstoffe zu verwendenden Arylhydrazone sind durch die Formel (V) allgemein definiert. In der Formel (V) haben R³, R⁴ und R⁵ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R³, R⁴ und R⁵ angegeben wurden.

Die Ausgangsstoffe der Formel (V) sind bekannt und/oder können nach bekannten Verfahren hergestellt werden (vgl. WO 86/00072, US 4956004).

Die beim erfindungsgemäßen Verfahren (c) weiter als Ausgangsstoffe zu verwendenden Alkalimetall(thio)cyanate sind durch die Formel (VI) allgemein definiert. In der Formel (VI) hat Q¹ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Q¹ angegeben wurde; M steht vorzugsweise für Lithium, Natrium, Kalium, Rubidium oder Cäsium, insbesondere für Natrium oder Kalium.

Die Ausgangsstoffe der Formel (VI) sind bekannte Synthesechemikalien.

Die beim erfindungsgemäßen Verfahren (c) weiter als Ausgangsstoffe zu verwendenden Ketocarbonsäuren sind durch die Formel (VIII) allgemein definiert. In der Formel (VIII) hat R² vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R² angegeben wurde.

Die Ausgangsstoffe der Formel (VIII) sind bekannte organische Synthesechemikalien.

Die bei den erfindungsgemäßen Verfahren (d) und (f) zur Herstellung von Verbindungen der Formel (I) als Ausgangsstoffe zu verwendenden Arylamine sind durch die Formel (IX) allgemein definiert. In der Formel (IX) haben R³, R⁴ und R⁵ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R³, R⁴ und R⁵ angegeben wurden.

Die Ausgangsstoffe der Formel (IX) sind bekannt und/oder können nach bekannten Verfahren hergestellt werden (vgl. EP 224001, EP 303573, DE 3835168, EP 403891).

Die beim erfindungsgemäßen Verfahren (d) weiter als Ausgangsstoffe zu verwendenden Cyanacetylcarbamidsäureester sind durch die Formel (X) allgemein definiert. In der Formel (X) hat R¹ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R¹ angegeben wurde.

Die Ausgangsstoffe der Formel (X) sind bekannte organische Synthesechemikalien.

Die beim erfindungsgemäßen Verfahren (e) zur Herstellung von Verbindungen der Formel (I) als Ausgangsstoffe zu verwendenden Arylhydrazine sind durch die Formel (XII) allgemein definiert. In der Formel (XII) haben R³, R⁴ und R⁵ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R³, R⁴ und R⁵ angegeben wurden.

Die Ausgangsstoffe der Formel (XII) sind bekannt und/oder können nach bekannten Verfahren hergestellt werden (vgl. EP 370332).

Die beim erfindungsgemäßen Verfahren (e) weiter als Ausgangsstoffe zu verwendenden Ketocarbonsäure-Derivate sind durch die Formel (XIII) allgemein definiert. In der Formel (XIII) hat R² vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R² angegeben wurde; R steht vorzugsweise für Wasserstoff oder C₁-C₄-Alkyl, insbesondere für Wasserstoff, Methyl oder Ethyl.

Die Ausgangsstoffe der Formel (XIII) sind bekannte organische Synthesechemikalien.

Die beim erfindungsgemäßen Verfahren (e) weiter als Ausgangsstoffe zu verwendenden Iso(thio)cyanate sind durch die Formel (XV) allgemein definiert. In der Formel (XV) haben Q¹ und R¹ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q¹ und R¹ angegeben wurden.

Die Ausgangsstoffe der Formel (XV) sind bekannte Synthesechemikalien.

Das beim erfindungsgemäßen Verfahren als Ausgangsstoff zu verwendende Malonyldiurethan der Formel (XVII) ist eine bekannte Synthesechemikalie.

Als Verdünnungsmittel zur Durchführung der erfindungsgemäßen Verfahren (a) bis (f) kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Als Reaktionshilfsmittel für die Verfahren (a) bis (f) kommen im allgemeinen die üblichen anorganischen oder organischen Basen oder Säureakzeptoren in Betracht. Hierzu gehören vorzugsweise Alkalimetall- oder Erdalkalimetall- -acetate, -amide, - carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- - methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die Reaktionstemperaturen können bei der Durchführung der erfindungsgemäßen Verfahren (a) bis (f) in einem größeren Bereich variiert werden. Im allgemeinen arbeitet man bei Temperaturen zwischen -20°C und +150°C, vorzugsweise zwischen 0°C und +120°C.

Die erfindungsgemäßen Verfahren (a) bis (f) werden im allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 10 bar - durchzuführen.

Zur Durchführung der erfindungsgemäßen Verfahren (a) bis (f) werden die Ausgangsstoffe im allgemeinen in angenähert äquimolaren Mengen eingesetzt. Es ist jedoch auch möglich, jeweils eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1:

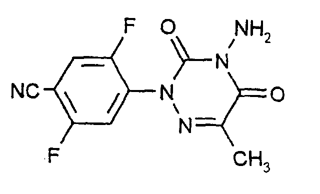

7,1 g (50 mMol) 4-Amino-6-methyl-1,2,4-triazin-3,5(2H,4H)-dion werden mit 6,9 g (50 mMol) Kaliumcarbonat und 7,9 g (50 mMol) 2,4,5-Trifluor-benzonitril in 100 ml Dimethylsulfoxid 4 Stunden bei 70°C gerührt, dann auf Raumtemperatur abgekühlt, auf Eiswasser ausgerührt, ausgefallenes Produkt abgesaugt, getrocknet und aus Ethanol umkristallisiert.

Man erhält 2,9 g (20 % der Theorie) 2-(2,4-Difluor-4-cyano-phenyl)-4-amino-4-methyl-1,2,4-triazin-3,5(2H,4H)-dion vom Schmelzpunkt 125°C.

### Beispiel 2:

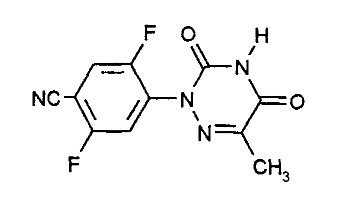

2,54 g (20 mMol) 6-Methyl-1,2,4-triazin-3,5(2H,4H)-dion werden in 50 ml Dimethylsulfoxyd mit 2,8 g (20 mMol) Kaliumcarbonat und 3,14 g (20 mol) 2,4,5-Trifluor-benzonitril 12 Stunden bei 120°C gerührt, dann auf Raumtemperatur abgekühlt, mit Wasser verührt, mit Salzsäure auf pH 5 eingestellt, ausgefallenes Produkt abgesaugt, mit Wasser gewaschen und getrocknet.

Man erhält 4,6 g (87% der Theorie) 2-(2,4-Difluor-4-cyano-phenyl)-6-methyl-1,2,4-triazin-3,5(2H,4H)-dion vom Schmelzpunkt 214°C.

### Beispiel 3:

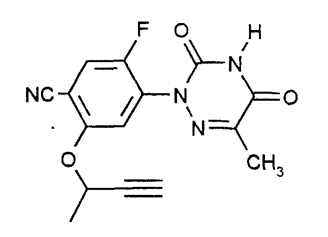

5,2 g (20 mMol) 2-(2,4-Difluor-4-cyano-phenyl)-6-methyl-1,2,4-triazin-3,5(2H,4H)-dion werden in 50 ml Acetonitril mit 2,8 g (40 mMol) 3-Butin-2-ol vorgelegt und bei Raumtemperatur (ca. 20°C) portionsweise mit 1,15 g (40 mMol) Natriumhydrid (80%ig in Paraffin) versetzt und 12 Stunden bei Raumtemperatur gerührt. Die Mischung wird unter vermindertem Druck eingeengt, der Rückstand mit Wasser verrührt, mit konz. Salzsäure auf pH 5 eingestellt und das ausgefallene Produkt durch Filtration isoliert.

Man erhält 5,8 g (92 % der Theorie) 2-(2-Fluor-4-cyano-5-but-1-in-3-yl-oxy-phenyl)-6-methyl-1,2,4-triazin-3,5(2H,4H)-dion vom Schmelzpunkt 153°C (Zers.).

### Beispiel 4:

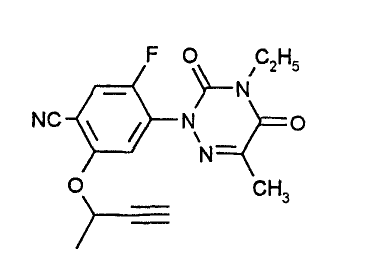

1,6 g (5 mMol) 2-(2-Fluor-4-cyano-5-but-1-in-3-yl-oxy-phenyl)-6-methyl-1,2,4-triazin-3,5(2H,4H)-dion werden in 50 ml Dimethylsulfoxyd mit 1,38 g (10 mMol) Kaliumcarbonat und 1,56 g (10 mMol) Ethyliodid 12 Stunden bei Raumtemperatur (ca. 20°C) gerührt. Die Mischung wird unter vermindertem Druck eingeengt, der Rückstand mit Wasser verrührt, mit konz. Salzsäure auf pH 5 eingestellt und das ausgefallene Produkt durch Filtration isoliert.

Man erhält 1,6 g (93,5 % der Theorie) 2-(2-Fluor-4-cyano-5-but-1-in-3-yl-oxyphenyl)-4-ethyl-6-methyl-1,2,4-triazin-3,5(2H,4H)-dion vom Schmelzpunkt 139°C.

Analog zu den Herstellungsbeispielen 1 bis 4 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

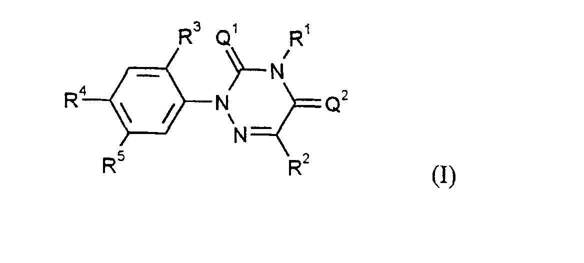

**Tabelle 1:**

| Beispiele für die Verbindungen der allgemeinen Formel (I) | | | | | | | | |
|---|---|---|---|---|---|---|---|---|
| Bsp. Nr. | R¹ | R² | Q¹ | Q² | R³ | R⁴ | R⁵ | physikal. Daten |
| 5 | NH₂ | C(CH₃)₃ | O | O | F | CN | F | Fp.: 120°C |
| 6 | H | H | O | O | F | CN | F | Fp.: >250°C |
| 8 | CH₃ | CH₃ | O | O | F | CN | OCH(CH₃)C≡CH | Fp.: 102°C |
| 9 | H | H | O | O | F | CN | OCH(CH₃)C≡CH | Fp.: 202°C |
| 10 | CH₃ | H | O | O | F | CN | OCH(CH₃)C≡CH | Fp.: 129°C |
| 11 | H | H | O | O | F | CN | NHSO₂C₂H₅ | Fp.: 121°C (Zers.) |
| 12 | H | H | O | O | F | CN | OCH₂-C≡CH | Fp.: 175°C |
| 13 | CH₃ | H | O | O | F | CN | OCH₂-C≡CH | Fp:: 137°C |
| 14 | CH₃ | H | O | O | F | CN | NHSO₂C₂H₅ [enth. ca. 30% -N(CH₃)-] | Fp.: 187°C |
| 15 | CH₃ | H | O | O | F | CN | OCH₂CH=CH₂ | Fp.:139°C |
| 16 | CH(CH₃)₂ | CH₃ | O | O | F | CN | OCH(CH₃)C≡CH | Fp.: 112°C |
| 18 | C₂H₅ | H | O | O | F | CN | OCH₂-C≡CH | ¹H-NMR: |
| 19 | C₂H₅ | H | O | O | F | CN | OCH(CH₃)C≡CH | Fp.: 132°C |
| 20 | CH(CH₃)₂ | H | O | O | F | CN | OCH(CH₃)C≡CH | Fp.: 70°C |
| 23 | CH₂CH₂F | H | O | O | F | CN | OCH(CH₃)C≡CH | |
| 24 | (CH₂)₃F | H | O | O | F | CN | OCH(CH₃)C≡CH | |
| 25 | CH₂-C≡CH | H | O | O | F | CN | OCH(CH₃)C≡CH | Fp.: 132°C |
| 26 | CH₂CH₂F | CH₃ | O | O | F | CN | OCH(CH₃)C≡CH | Fp.: 119°C |
| 27 | (CH₂)₃F | CH₃ | O | O | F | CN | OCH(CH₃)C≡CH | Fp.: 115°C |
| 28 | CH₃ | CH₃ | O | O | F | CN | NHSO₂C₂H₅ | Fp.: 129°C |
| 29 | CH₂CH=CH₂ | H | O | O | F | CN | OCH(CH₃)C≡CH | Fp.: 84°C |
| 29 | CH₂CH=CH₂ | H | O | O | F | CN | OCH(CH₃)C≡CH | Fp.: 84°C |
| 30 | CH₃ | CH₃ | O | O | F | CN | NHSO₂CH₃ | Fp.: 115°C |
| 31 | (CH₂)₃F | H | O | O | F | CN | F | Fp.: 85°C |
| 32 | H | CH₃ | O | O | F | CN | NHSO₂CH₃ | Fp.: 140° |
| 33 | (CH₂)₃F | CH₃ | O | O | F | CN | NHSO₂CH₃ | Fp.: 108°C |
| 34 | CH₃ | H | O | S | F | CN | OCH(CH₃)C≡CH | (amorph) |
| 35 | CH₂CH₂F | H | O | O | F | CN | OCH₂C≡CH | Fp.: 135°C |
| 36 | (CH₂)₂CF=CF₂ | H | O | O | F | CN | OCH₂C≡CH | (amorph) |
| 37 | (CH₂)₃F | H | O | O | F | CN | OCH₂C≡CH | Fp.: 126°C |
| 38 | (CH₂)₂CF=CF₂ | H | O | O | F | CN | NHSO₂C₂H₅ | Fp.: 88°C |
| 39 | (CH₂)₃F | CH₃ | O | O | F | CSNH₂ | NHSO₂CH₃ | Fp.: >210°C |
| 40 | C₂H₅ | H | O | O | F | CN | N(C₂H₅)SO₂C₂H₅ | Fp.: 154°C |
| 41 | CH₃ | H | O | O | F | CN | N(CH₃)SO₂C₂H₅ | Fp.: 91°C |
| 42 | H | H | O | O | F | CN | N(SO₂C₂H₅)₂ | Fp.: >250°C |
| 43 | CH₃ | H | O | O | F | CN | N(SO₂C₂H₅)₂ | Fp.: 164°C |
| 44 | CH₃ | H | O | O | F | CN | NHSO₂C₂H₅ | Fp.: 233°C |
| 45 | CH₂CH₂F | H | O | O | F | CN | NHSO₂C₂H₅ | Fp.: 190°C |
| 46 | (CH₂)₃F | H | O | O | F | CN | NHSO₂C₂H₅ | Fp.: 98°C |
| 47 | (CH₂)₃F | H | O | O | F | CN | NHSO₂CH₃ | Fp.: 186°C |
| 48 | CH₂CH₂F | H | O | O | F | CN | NHSO₂CH₃ | Fp.: 178°C |

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

In diesem Test zeigen beispielsweise die Verbindungen gemäß Herstellungsbeispiel 7, 8, 10, 17, 18 und 19 bei Aufwandmenge zwischen 60 und 125 g/ha und guter Verträglichkeit gegenüber Kulturpflanzen, wie z.B. Weizen (bis 20 %), Gerste (0 bis 10 %), Mais (0 %), Soja (10 bis 20 %) und Baumwolle (0 bis 30 %), sehr starke Wirkung gegen Unkräuter wie Alopecurus (70 bis 100 %), Digitaria (70 bis 100 %), Sorghum (70 bis 100 %), Amaranthus (100 %), Chenopodium (100 %) sowie Solanum (100 %).

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

In diesem Test zeigen beispielsweise die Verbindungen gemäß Herstellungsbeispiel 7, 8 und 10 bei Auswandmengen zwischen 60 und 125 g/ha und guter Verträglichkeit gegenüber Kulturpflanzen, wie z.B. Weizen (0 bis 10 %) und Gerste (0 %), sehr starke Wirkung gegen Unkräuter wie Digitaria (70 bis 95 %), Echinochloa (80 bis 100 %), Setaria (70 bis 100 %), Datura (100 %), Solanum (100 %) sowie Viola (100 %).

## Patentansprüche

1. Substituierte 2-Aryl-1,2,4-triazin-3,5-di(thi)one der allgemeinen Formel (I), 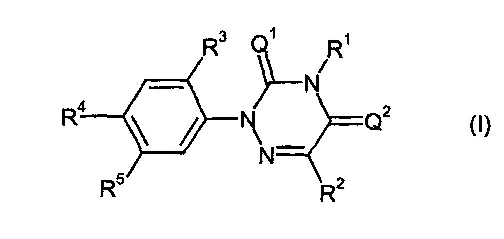 **dadurch gekennzeichnet, dass**
Q¹ für Sauerstoff oder Schwefel steht,
Q² für Sauerstoff oder Schwefel steht,
R¹ für Wasserstoff, Cyano, Amino, für jeweils gegebenenfalls durch Cyano, Fluor, Chlor, C₁-C₄-Alkoxy oder C₁-C₄-Alkylthio substituiertes Alkyl, Alkoxy, Alkylamino, Dialkylamino, Alkylcarbonyl oder Alkoxycarbonyl mit jeweils 1 bis 6 Kohlenstoffatomen in den Alkylgruppen, für jeweils gegebenenfalls durch Fluor, Chlor oder Brom substituiertes Alkenyl, Alkenylcarbonyl, Alkenyloxycarbonyl, Alkinyl, Alkinylcarbonyl oder Alkinyloxycarbonyl mit jeweils 2 bis 6 Kohlenstoffatomen in den Alkenyl- bzw. Alkinyl-gruppen, oder fiir jeweils gegebenenfalls durch Cyano, Fluor, Chlor, Brom oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil steht,
R² für Wasserstoff, Halogen, Nitro, Carboxy, Cyano, Thiocarbamoyl, Amino, für jeweils gegebenenfalls durch Cyano, Fluor, Chlor, C₁-C₄-Alkoxy oder C₁-C₄-Alkylthio substituiertes Alkyl, Alkoxy, Alkylthio, Alkylamino oder Dialkylamino mit jeweils 1 bis 6 Kohlenstoffatomen in den Alkylgruppen, für jeweils gegebenenfalls durch Fluor, Chlor oder Brom substituiertes Alkenyl, Alkenyloxy, Alkenylthio, Alkinyl, Alkinyloxy oder Alkinylthio mit jeweils 2 bis 6 Kohlenstoffatomen in den Alkenyl- oder Alkinyl-gruppen, oder für jeweils gegebenenfalls durch Cyano, Fluor, Chlor, Brom oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 6 Kohlenstoffatomen im Alkylteil steht,
R³ für Fluor, Chlor oder Brom steht,
R⁴ für Cyano oder Thiocarbamoyl steht und
R⁵ für die Gruppierung -A¹-A²-A³ steht,
in welcher
A¹ für Sauerstoff steht,
A² für eine Einfachbindung steht, und
A³ für jeweils gegebenenfalls durch Fluor oder Chlor substituiertes Alkenyl oder Alkinyl mit jeweils 2 bis 6 Kohlenstoffatomen in den Alkenyl - oder Alkinylgruppen steht, oder
A¹ für die Gruppierung -N-A⁴- steht,
A² für eine Einfachbindung steht, und
A³ für gegebenenfalls durch Fluor, Chlor oder C₁-C₄-Alkoxy substituiertes Alkylsulfonyl mit 1 bis 6 Kohlenstoffatomen in den Alkylgruppen steht, und
A⁴ für Wasserstoff oder C₁-C₄-Alkylsulfonyl steht.

2. Verfahren zur Herstellung substituierter 2-Aryl-1,2,4-triazin-3,5-di(thi)one der allgemeinen Formel (I), 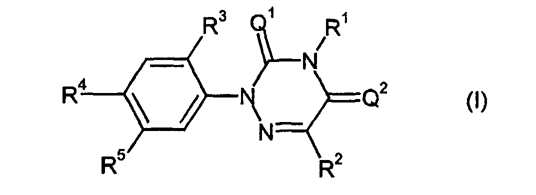 in welcher
R¹, R², R³, R⁴, R⁵, Q¹, und Q² die in Anspruch 1 genannten Bedeutungen haben, **dadurch gekennzeichnet, dass** man
(a) 1,2,4-Triazin-3,5-di(thi)one der allgemeinen Formel (II), 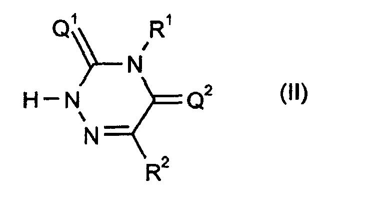 in welcher
Q¹, Q², R¹ und R² die oben angegebenen Bedeutungen haben,
mit Halogenbenzol-Derivaten der allgemeinen Formel (III), 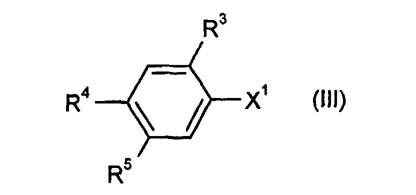 in welcher
R³, R⁴ und R⁵ die oben angegebenen Bedeutungen haben und
X¹ für Halogen steht,
gegebenenfalls in Gegenwart eines Verdünnungsmittels und gegebenenfalls in Gegenwart eines Reaktionshilfsmittels umsetzt,
oder dass man
(b) 2-Aryl-1,2,4-triazin-3,5-di(thi)one der allgemeinen Formel (Ia), 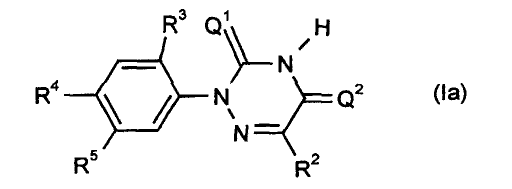 in welcher
Q¹, Q², R², R³, R⁴ und R⁵ die oben angegebenen Bedeutungen haben
mit Alkylierungsmitteln der allgemeinen Formel (IV),
R¹-X² (IV)
in welcher
R¹ die oben angegebene Bedeutung hat und
X² für Halogen oder die Gruppierung -O-SO₂-O-R¹ steht,
gegebenenfalls in Gegenwart eines Verdünnungsmittels und gegebenenfalls in Gegenwart eines Reaktionshilfsmittels umsetzt,
oder dass man
(c) Arylhydrazone der allgemeinen Formel (V), 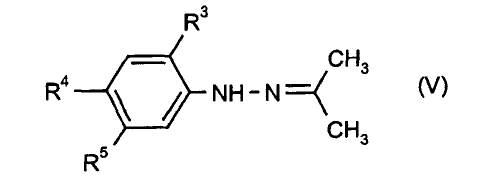 in welcher
R³, R⁴ und R⁵ die oben angegebenen Bedeutungen haben
mit Alkalimetall(thio)cyanaten der allgemeinen Formel (VI),
M-Q¹-CN (VI)
in welcher
Q¹ die oben angegebene Bedeutung hat und
M für ein Alkalimetallatom steht,
gegebenenfalls in Gegenwart eines Verdünnungsmittels und gegebenenfalls in Gegenwart eines Reaktionshilfsmittels umsetzt und die hierbei gebildeten 2-Aryl-1,2,4-triazolidin-3-(thi)one der allgemeinen Formel (VII), 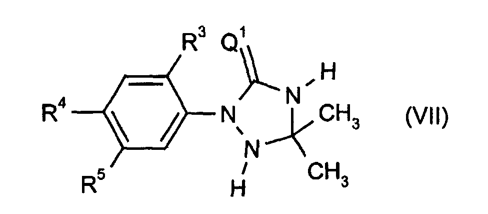 in welcher
Q¹, R³, R⁴ und R⁵ die oben angegebenen Bedeutungen haben
mit Ketocarbonsäuren der allgemeinen Formel (VIII), 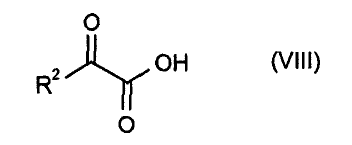 in welcher
R² die oben angegebene Bedeutung hat
gegebenenfalls in Gegenwart eines Verdünnungsmittels und gegebenenfalls in Gegenwart eines Reaktionshilfsmittels umsetzt,
oder dass man
(d) Aryl-amine der allgemeinen Formel (IX), 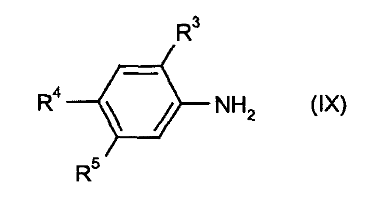 in welcher
R³, R⁴ und R⁵ die oben angegebenen Bedeutungen haben
diazotiert und im Anschluss daran mit einem Cyanacetylcarbamidsäureester der allgemeinen Formel (X), 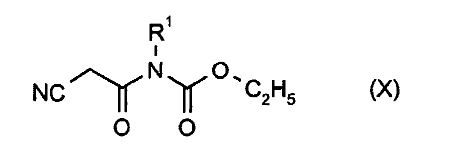 in welcher
R¹ die oben angegebene Bedeutung hat,
gegebenenfalls in Gegenwart eines Verdünnungsmittels und gegebenenfalls in Gegenwart eines Reaktionshilfsmittels umsetzt,
und die so erhaltenen Verbindungen der allgemeinen Formel (XI), 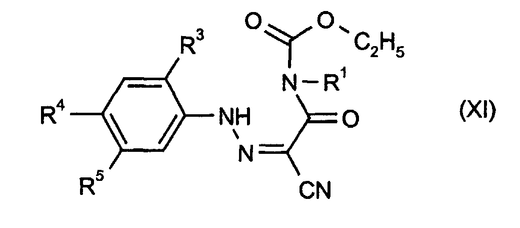 in welcher
R¹, R³, R⁴ und R⁵ die oben angegebenen Bedeutungen haben
gegebenenfalls in Gegenwart eines Verdünnungsmittels und gegebenenfalls in Gegenwart eines Reaktionshilfsmittels cyclisiert,
oder dass man
(e) Arylhydrazine der allgemeinen Formel (XII) 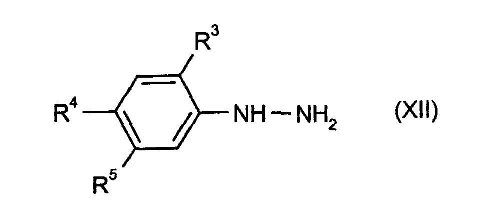 in welcher
R³, R⁴ und R⁵ die oben angegebenen Bedeutungen haben
mit Ketocarbonsäure-Derivaten der allgemeinen Formel (XIII), 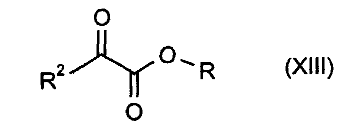 in welcher
R² die oben angegebene Bedeutung hat und
R für Wasserstoff oder Alkyl steht,
gegebenenfalls in Gegenwart eines Verdünnungsmittels und gegebenenfalls in Gegenwart eines Reaktionshilfsmittels umsetzt und die so erhaltenen Verbindungen der allgemeinen Formel (XIV), 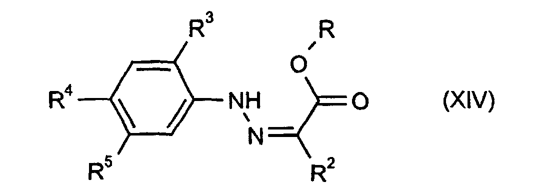 in welcher
R²,R³, R⁴ und R⁵ die oben angegebenen Bedeutungen haben und
R für Wasserstoff oder Alkyl steht,
gegebenenfalls in Gegenwart eines Verdünnungsmittels und gegebenenfalls in Gegenwart eines Reaktionshilfsmittels mit Iso(thio)cyanaten der allgemeinen Formel (XV),
R¹-N=C=Q¹ (XV)
in welcher
Q¹ und R¹ die oben angegebenen Bedeutungen haben,
gegebenenfalls in Gegenwart eines Verdünnungsmittels und gegebenenfalls in Gegenwart eines Reaktionshilfsmittels umsetzt
und die so erhaltenen Verbindungen der allgemeinen Formel (XVI), 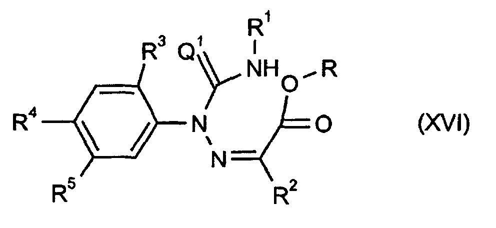 in welcher
Q¹, R¹, R², R³, R⁴ und R⁵ die oben angegebenen Bedeutungen haben und
R für Wasserstoff oder Alkyl steht,
gegebenenfalls in Gegenwart eines Verdünnungsmittels und gegebenenfalls in Gegenwart eines Reaktionshilfsmittels cyclisiert,
oder dass man
(f) 2-Aryl-1,2,4-triazin-3,5-dion-6-carbonsäuren der allgemeinen Formel (XIX), 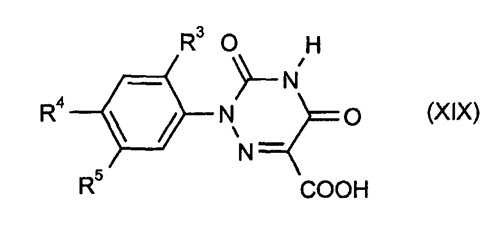 in welcher
R³, R⁴ und R⁵ die oben angegebenen Bedeutung haben erhält,
wenn man Aryl-amine der allgemeinen Formel (IX), 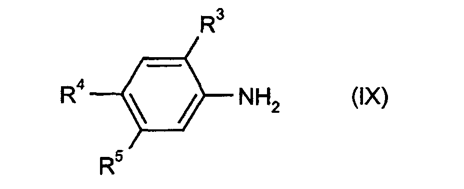 in welcher
R³, R⁴ und R⁵ die oben angegebenen Bedeutungen haben
diazotiert und im Anschluss daran mit dem Malonyldiurethan der Formel (XVII),
CH₂(CO-NH-COOC₂H₅)₂ (XVII)
gegebenenfalls in Gegenwart eines Verdünnungsmittels und gegebenenfalls in Gegenwart eines Reaktionshilfsmittels umsetzt und anschließend cyclisiert.

3. Substituierte 2-Aryl-1,2,4-triazin-3,5-di(thi)one der allgemeinen Formel (I) gemäß Anspruch 1, **dadurch gekennzeichnet, dass**
Q¹ für Sauerstoff oder Schwefel steht,
Q² für Sauerstoff oder Schwefel steht,
R¹ für Wasserstoff, Cyano, Amino, für jeweils gegebenenfalls durch Cyano, Fluor, Chlor, Methoxy oder Ethoxy substituiertes Methyl, Ethyl, n- oder i-Propyl, n-, i- s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino, Dimethylamino, Diethylamino, Acetyl, Propionyl, n- oder i-Butyroyl, Methoxycarbonyl, Ethoxycarbonyl, n- oder i-Propoxycarbonyl, für jeweils gegebenenfalls durch Fluor, Chlor oder Brom substituiertes Propenyl, Butenyl, Propenylcarbonyl, Butenylcarbonyl, Propenyloxycarbonyl, Butenyloxycarbonyl, für Propinyl, Butinyl, Propinylcarbonyl, Butinylcarbonyl, Propinyloxycarbonyl oder Butinyloxycarbonyl, oder für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R² für Wasserstoff, Halogen, Nitro, Carboxy, Cyano, Thiocarbamoyl, Amino, für jeweils gegebenenfalls durch Cyano, Fluor, Chlor, Methoxy oder Ethoxy substituiertes Methyl, Ethyl, n- oder i-Propyl, n-, i-s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino, für jeweils gegebenenfalls durch Fluor, Chlor oder Brom substituiertes Propenyl, Propenyloxy, Propenylthio, Butenyl, Butenyloxy oder Butenylthio, für Propinyl, Propinyloxy, Propinylthio, Butinyl, Butinyloxy oder Butinylthio, oder für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R³ für Fluor oder Chlor steht,
R⁴ für Cyano oder Thiocarbamoyl steht und
R⁵ für die Gruppierung -A¹-A²-A³ steht,
in welcher
A¹ für Sauerstoff,
A² für eine Einfachbindung, und
A³ für jeweils gegebenenfalls durch Fluor oder Chlor substituiertes Propenyl, Butenyl, Propinyl, Butinyl steht, oder
A¹ für die Gruppierung -NH-,
A² für eine Einfachbindung, und
A³ für jeweils gegebenenfalls durch Fluor, Chlor, Methoxy oder Ethoxy substituiertes Methylsulfonyl, Ethylsulfonyl, n- oder i-Propylsulfonyl steht.

4. Herbizide Mittel, **gekennzeichnet durch** einen Gehalt an mindestens einem substituierten 2-Aryl-1,2,4-triazin-3,5-di(thi)on der allgemeinen Formel (I) gemäß den Ansprüchen 1 oder 3.

5. Verfahren zur Bekämpfung von unerwünschten Pflanzen, **dadurch gekennzeichnet, dass** man substituierte 2-Aryl-1,2,4-triazin-3,5-di(thi)one der allgemeinen Formel (I) gemäß den Ansprüchen 1 oder 3 auf unerwünschte Pflanzen und/oder ihre Lebensräume einwirken lässt.

6. Verwendung von substituierten 2-Aryl-1,2,4-triazin-3,5-di(thi)onen der allgemeinen Formel (I) gemäß den Ansprüchen 1 oder 3, zur Bekämpfung von unerwünschten Pflanzen.

7. Verfahren zur Herstellung von herbiziden Mitteln, **dadurch gekennzeichnet, dass** man substituierte 2-Aryl-1,2,4-triazin-3,5-di(thi)one der allgemeinen Formel (I) gemäß den Ansprüchen 1 oder 3 mit Streckmitteln und/oder oberflächenaktiven Substanzen vermischt.

8. Substituierte 2-Aryl-1,2,4-triazin-3,5-di(thi)one der allgemeinen Formel (I), 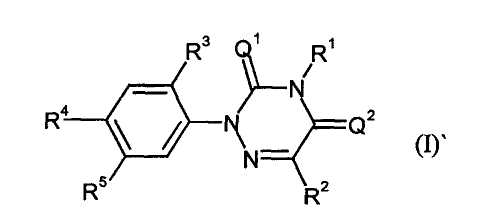 **dadurch gekennzeichnet, dass**
Q¹ für Sauerstoff oder Schwefel steht,
Q² für Sauerstoff oder Schwefel steht,
R¹ für Wasserstoff, Cyano, Amino, für jeweils gegebenenfalls durch Cyano, Fluor, Chlor, C₁-C₄-Alkoxy oder C₁-C₄-Alkylthio substituiertes Alkyl, Alkoxy, Alkylamino, Dialkylamino, Alkylcarbonyl oder Alkoxycarbonyl mit jeweils 1 bis 6 Kohlenstoffatomen in den Alkylgruppen, für jeweils gegebenenfalls durch Fluor, Chlor oder Brom substituiertes Alkenyl, Alkenylcarbonyl, Alkenyloxycarbonyl, Alkinyl, Alkinylcarbonyl oder Alkinyloxycarbonyl mit jeweils 2 bis 6 Kohlenstoffatomen in den Alkenyl- bzw. Alkinyl-gruppen, oder für jeweils gegebenenfalls durch Cyano, Fluor, Chlor, Brom oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil steht,
R² für Wasserstoff, Halogen, Nitro, Carboxy, Cyano, Thiocarbamoyl, Amino, für jeweils gegebenenfalls durch Cyano, Fluor, Chlor, C₁-C₄-Alkoxy oder C₁-C₄-Alkylthio substituiertes Alkyl, Alkoxy, Alkylthio, Alkylamino oder Dialkylamino mit jeweils 1 bis 6 Kohlenstoffatomen in den Alkylgruppen, für jeweils gegebenenfalls durch Fluor, Chlor oder Brom substituiertes Alkenyl, Alkenyloxy, Alkenylthio, Alkinyl, Alkinyloxy oder Alkinylthio mit jeweils 2 bis 6 Kohlenstoffatomen in den Alkenyl- oder Alkinyl-gruppen, oder für jeweils gegebenenfalls durch Cyano, Fluor, Chlor, Brom oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 6 Kohlenstoffatomen im Alkylteil steht,
R³ für Fluor, Chlor oder Brom steht,
R⁴ für Cyano oder Thiocarbamoyl steht und
R⁵ für Amino oder Fluor steht.

9. Verbindungen der Formel (I) gemäß Anspruch 1, bei denen die in der folgenden Tabelle angegebenen Restedefinitionen vorliegen:
| R¹ | R² | Q¹ | Q² | R³ | R⁴ | R⁵ |
|---|---|---|---|---|---|---|
| CH₃ | CH₃ | O | O | F | CN | OCH(CH₃)C≡CH |
| CH₃ | H | O | O | F | CN | OCH(CH₃)C≡CH |
| (CH₂)₂F | H | O | O | F | CN | OCH(CH₃)C≡CH |
| C₂H₅ | H | O | O | F | CN | N(C₂H₅)SO₂C₂H₅ |
| CH₃ | H | O | O | F | CN | N(SO₂C₂H₅)₂ |
| CH₃ | H | O | O | F | CN | NHSO₂C₂H₅ |

## Claims

1. Substituted 2-aryl-1,2,4-triazine-3,5-di(thi)ones of the general formula (I), 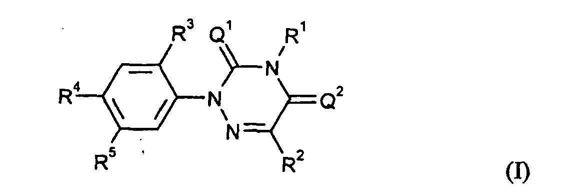 **characterized in that**
Q¹ represents oxygen or sulphur,
Q² represents oxygen or sulphur,
R¹ represents hydrogen, cyano, amino, represents in each case optionally cyano-, fluorine-, chlorine-, C₁-C₄-alkoxy- or C₁-C₄-alkylthio-substituted alky, alkoxy, alkylamino, dialkylamino, alkylcarbonyl or alkoxycarbonyl having in each case 1 to 6 carbon atoms in the alkyl groups, represents in each case optionally fluorine-, chlorine- or bromine-substituted alkenyl, alkenylcarbonyl, alkenyloxycarbonyl, alkinyl, alkinylcarbonyl or alkinyloxycarbonyl having in each case 2 to 6 carbon atoms in the alkenyl or alkinyl group, or represents in each case optionally cyano-, fluorine-, chlorine-, bromine- or C₁-C₄-alkyl-substituted cycloalkyl or cycloalkylalkyl having in each case 3 to 6 carbon atoms in the cycloalkyl groups and, if appropriate, 1 to 4 carbon atoms in the alkyl moiety,
R² represents hydrogen, halogen, nitro, carboxyl, cyano, thiocarbamoyl, amino, represents in each case optionally cyano-, fluorine-, chlorine-, C₁-C₄-alkoxy- or C₁-C₄-alkylthio-substituted alkyl, alkoxy, alkylthio, alkylamino or dialkylamino having in each case 1 to 6 carbon atoms in the alkyl groups, represents in each case optionally fluorine-, chlorine- or bromine-substituted alkenyl, alkenyloxy, alkenylthio, alkinyl, alkinyloxy or alkinylthio having in each case 2 to 6 carbon atoms in the alkenyl or alkinyl groups, or represents in each case optionally cyano-, fluorine-, chlorine-, bromine- or C₁-C₄-alkyl-substituted cycloalkyl or cycloalkylalkyl having in each case 3 to 6 carbon atoms in the cycloalkyl groups and, if appropriate, 1 to 6 carbon atoms in the alkyl moiety,
R³ represents fluorine, chlorine or bromine,
R⁴ represents cyano or thiocarbamoyl and
R⁵ represents the grouping -A¹-A²-A³
in which
A¹ represents represents oxygen,
A² represents a single bond,
A³ represents optionally fluorine-, chlorine- or C₁-C₄-alkoxy-substituted alkylsulphonyl having 1 to 6 carbon atoms in the alkyl groups, and
A⁴ represents hydrogen or C₁-C₄-alkylsulphonyl.

2. Process for preparing substituted 2-aryl-1,2,4-triazine-3,5-di(thi)one of the general formula (I), 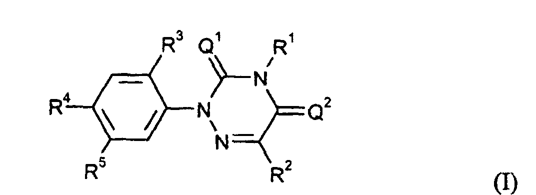 in which
R¹, R², R³, R⁴, R⁵, Q¹, and Q² are each as defined in Claim 1, **characterized in that**
(a) 1,2,4-triazine-3,5-di(thi)ones of the general formula (II), 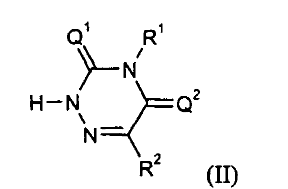 in which
Q¹, Q², R¹ and R² are each as defined above,
are reacted with halogenobenzene derivatives of the general formula (III), 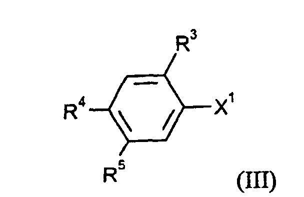 in which
R³, R⁴ and R⁵ are each as defined above and
X¹ represents halogen,
if appropriate in the presence of a diluent and if appropriate in the presence of a reaction auxiliary,
or that
(b) 2-aryl-1,2,4-triazine-3,5-di(thi)ones of the general formula (Ia), 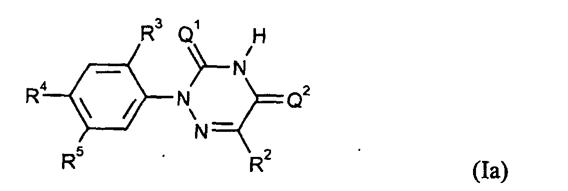 in which
Q¹, Q², R², R³, R⁴ and R⁵ are each as defined above
are reacted with alkylating agents of the general formula (IV),
R¹-X² (IV)
in which
R¹ is as defined above and
X² represents halogen or the grouping -O-SO₂-O-R¹,
if appropriate in the presence of a diluent and if appropriate in the presence of a reaction auxiliary,
or that
(c) arylhydrazones of the general formula (V), 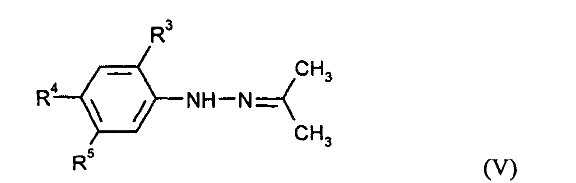 in which
R³, R⁴ and R⁵ are each as defined above
are reacted with alkali metal (thio)cyanates of the general formula (VI),
M-Q¹-CN (VI)
in which
Q¹ is as defined above and
M represents an alkali metal atom, if appropriate in the presence of a diluent and if appropriate in the presence of a reaction auxiliary and the resulting 2-aryl-1,2,4-triazolidine-3-(thi)ones of the general formula (VII), 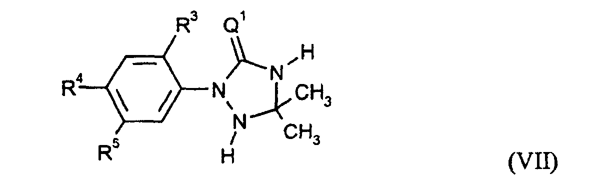 in which
Q^{1,} R³, R⁴ and R⁵ are each as defined above
are reacted with ketocarboxylic acids of the general formula (VIII), 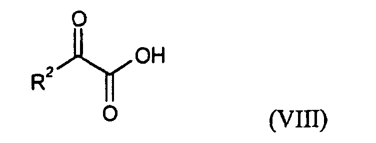 in which
R² is as defined above
if appropriate in the presence of a diluent and if appropriate in the presence of a reaction auxiliary,
or that
(d) aryl-amines of the general formula (IX), 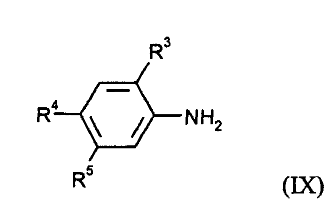 in which
R³, R⁴ and R⁵ are each as defined above
are diazotized and subsequently reacted with a cyanoacetylcarbamic acid ester of the general formula (X), 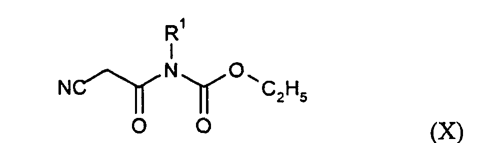 in which
R¹ is as defined above,
if appropriate in the presence of a diluent and if appropriate in the presence of a reaction auxiliary,
and the resulting compounds of the general formula (XI), 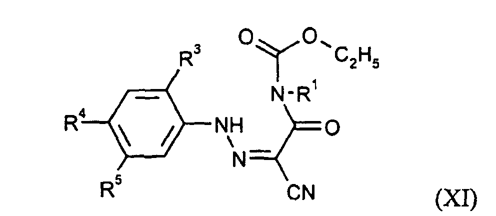 in which
R¹, R³, R⁴ and R⁵ are each as defined above
are cyclized if appropriate in the presence of a diluent and if appropriate in the presence of a reaction auxiliary,
or that
(e) arylhydrazines of the general formula (XII) 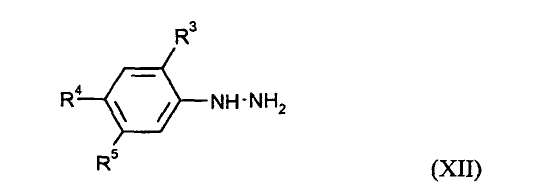 in which
R³, R⁴ and R⁵ are each as defined above
are reacted with ketocarboxylic acid derivatives of the general formula (XIII), 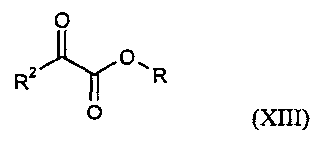 in which
R² is as defined above and
R represents hydrogen or alkyl,
if appropriate in the presence of a diluent and if appropriate in the presence of a reaction auxiliary, and the resulting compounds of the general formula (XIV) 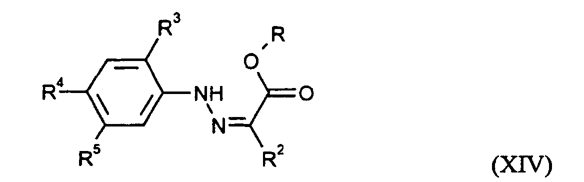 in which
R²,R³, R⁴ and R⁵ are each as defined above and
R represents hydrogen or alkyl,
are reacted, if appropriate in the presence of a diluent and if appropriate in the presence of a reaction auxiliary, with iso(thio)cyanates of the general formula (XV),
R¹-N=C=Q¹ (XV)
in which
Q¹ and R¹ are each as defined above,
if appropriate in the presence of a diluent and if appropriate in the presence of a reaction auxiliary
and the resulting compounds of the general formula (XVI), 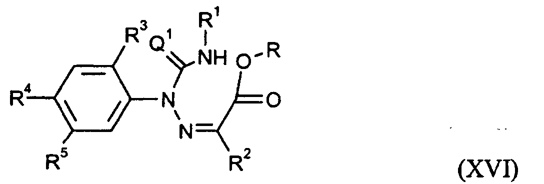 in which
Q¹, R¹, R², R³, R⁴ and R⁵ are each as defined above and
R represents hydrogen or alkyl,
are cyclized, if appropriate in the presence of a diluent and if appropriate in the presence of a reaction auxiliary,
or that
(f) 2-aryl-1,2,4-triazine-3,5-dione-6-carboxylic acids of the general formula (XIX), 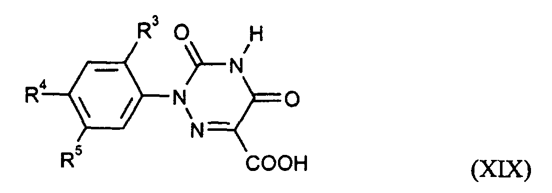 in which
R³, R⁴ and R⁵ are each as defined above are obtained,
if aryl-amines of the general formula (IX), 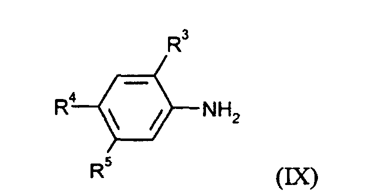 in which
R³, R⁴ and R⁵ are each as defined above
are diazotized and subsequently reacted with the malonyldiurethane of the formula (XVII),
CH₂(CO-NH-COOC₂H₅)₂ (XVII)
if appropriate in the presence of a diluent and if appropriate in the presence of a reaction auxiliary and subsequently cyclized.

3. Substituted 2-aryl-1,2,4-triazine-3,5-di(thi)ones of the general formula (I) according to Claim 1, **characterized in that**
Q¹ represents oxygen or sulphur,
Q² represents oxygen or sulphur,
R¹ represents hydrogen, cyano, amino, represents respectively optionally cyano-, fluorine-, chlorine-, methoxy- or ethoxy-substituted methyl, ethyl, n- or i-propyl, n-, i- s- or t-butyl, methoxy, ethoxy, n- or i-propoxy, n-, i-, s- or t-butoxy, methylamino, ethylamino, n- or i-propylamino, n-, i-, s- or t-butylamino, dimethylamino, diethylamino, acetyl, propionyl, n- or i-butyroyl, methoxycarbonyl, ethoxycarbonyl, n- or i-propoxycarbonyl, represents respectively optionally fluorine-, chlorine- or bromine-substituted propenyl, butenyl, propenylcarbonyl, butenylcarbonyl, propenyloxycarbonyl, butenyloxycarbonyl, represents propinyl, butinyl, propinylcarbonyl, butinylcarbonyl, propinyloxycarbonyl or butinyloxycarbonyl, or represents respectively optionally cyano-, fluorine-, chlorine-, bromine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl,
R² represents hydrogen, halogen, nitro, carboxyl, cyano, thiocarbamoyl, amino, represents respectively optionally cyano-, fluorine-, chlorine-, methoxy- or ethoxy-substituted methyl, ethyl, n- or i-propyl, n-, i- s- or t-butyl, methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, dimethylamino or diethylamino, represents respectively optionally fluorine-, chlorine- or bromine-substituted propenyl, propenyloxy, propenylthio, butenyl, butenyloxy or butenylthio, represents propinyl, propinyloxy, propinylthio, butinyl, butinyloxy or butinylthio, or represents respectively optionally cyano-, fluorine-, chlorine-, bromine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl,
R³ represents fluorine or chlorine,
R⁴ represents cyano or thiocarbamoyl and
R⁵ represents the grouping -A¹-A²-A³,
in which
A¹ represents oxygen,
A² represents a single bond, and
A³ represents in each case optionally fluorine- or chlorine-substituted propenyl, butenyl, propinyl, butinyl, or
A¹ represents the grouping -NH-,
A² represents a single bond, and
A³ represents in each case optionally fluorine-, chlorine-, methoxy- or ethoxy-substituted methylsulphonyl, ethylsulphonyl, n- or i-propylsulphonyl.

4. Herbicidal compositions, **characterized in that** they comprise at least one substituted 2-aryl-1,2,4-triazine-3,5-di(thi)one of the general formula (I) according to Claim 1 or 3.

5. Method for controlling unwanted plants, **characterized in that** substituted 2-aryl-1,2,4-triazine-3,5-di(thi)ones of the general formula (I) according to Claim 1 or 3 are allowed to act on unwanted plants and/or their habitats.

6. Use of substituted 2-aryl-1,2,4-triazine-3,5-di(thi)ones of the general formula (I) according to Claim 1 or 3, for controlling unwanted plants.

7. Process for preparing herbicidal compositions, **characterized in that** substituted 2-aryl-1,2,4-triazine-3,5-di(thi)ones of the general formula (I) according to Claim 1 or 3 are mixed with extenders and/or surfactants.

8. Substituted 2-aryl-1,2,4-triazine-3,5-di(thi)ones of the general formula (I)', 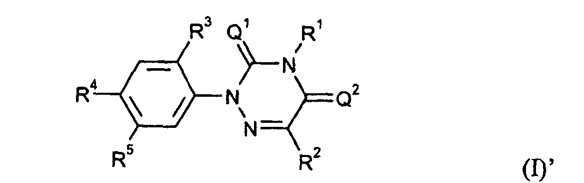 **characterized in that**
Q¹ represents oxygen or sulphur,
Q² represents oxygen or sulphur,
R¹ represents hydrogen, cyano, amino, represents in each case optionally cyano-, fluorine-, chlorine-, C₁-C₄-alkoxy- or C₁-C₄-alkylthio-substituted alkyl, alkoxy, alkylamino, dialkylamino, alkylcarbonyl or alkoxycarbonyl having in each case 1 to 6 carbon atoms in the alkyl groups, represents in each case optionally fluorine-, chlorine- or bromine-substituted alkenyl, alkenylcarbonyl, alkenyloxycarbonyl, alkinyl, alkinylcarbonyl or alkinyloxycarbonyl having in each case 2 to 6 carbon atoms in the alkenyl or alkinyl group, or represents in each case optionally cyano-, fluorine-, chlorine-, bromine- or C₁-C₄-alkyl-substituted cycloalkyl or cycloalkylalkyl having in each case 3 to 6 carbon atoms in the cycloalkyl groups and, if appropriate, 1 to 4 carbon atoms in the alkyl moiety,
R² represents hydrogen, halogen, nitro, carboxyl, cyano, thiocarbamoyl, amino, represents in each case optionally cyano-, fluorine-, chlorine-, C₁-C₄-alkoxy- or C₁-C₄-alkylthio-substituted alkyl, alkoxy, alkylthio, alkylamino or dialkylamino having in each case 1 to 6 carbon atoms in the alkyl groups, represents in each case optionally fluorine-, chlorine- or bromine-substituted alkenyl, alkenyloxy, alkenylthio, alkinyl, alkinyloxy or alkinylthio having in each case 2 to 6 carbon atoms in the alkenyl or alkinyl groups, or represents in each case optionally cyano-, fluorine-, chlorine-, bromine- or C₁-C₄-alkyl-substituted cycloalkyl or cycloalkylalkyl having in each case 3 to 6 carbon atoms in the cycloalkyl groups and, if appropriate, 1 to 6 carbon atoms in the alkyl moiety,
R³ represents fluorine, chlorine or bromine,
R⁴ represents cyano or thiocarbamoyl and
R⁵ represents amino or fluorine.

9. Compounds of the formula (I) according to Claim 1 in which the radical definitions given in the table below apply:
| R¹ | R² | Q¹ | Q² | R³ | R⁴ | R⁵ |
|---|---|---|---|---|---|---|
| CH₃ | CH₃ | O | O | F | CN | OCH(CH₃)C=CH |
| CH₃ | H | O | O | F | CN | OCH(CH₃)C=CH |
| (CH₂)₂F | H | O | O | F | CN | OCH(CH₃)C=CH |
| C₂H₅ | H | O | O | F | CN | N(C₂H₅)SO₂C₂H₅ |
| CH₃ | H | O | O | F | CN | N(SO₂C₂H₅)₂ |
| CH₃ | H | O | O | F | CN | NHSO₂C₂H₅ |

## Revendications

1. 2-aryl-1,2,4-triazine-3,5-di(thi)ones substituées de formule générale (I) 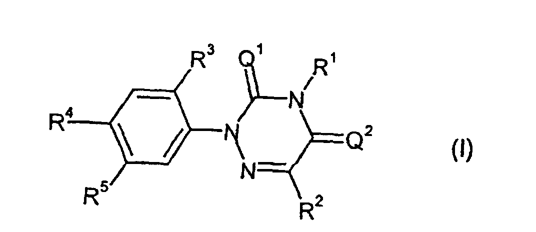 **caractérisées en ce que**
Q¹ représente l'oxygène ou le soufre,
Q² représente l'oxygène ou le soufre,
R¹ est l'hydrogène, un reste cyano, amino, un reste alkyle, alkoxy, alkylamino, dialkylamino, alkyl-carbonyle ou alkoxycarbonyle ayant 1 à 6 atomes de carbone dans les groupes alkyle et portant chacun, le cas échéant, un substituant cyano, fluoro, chloro, alkoxy en C₁ à C₄ ou alkylthio en C₁ à C₄, un reste alcényle, alcénylcarbonyle, alcényloxycarbonyle, alcynyle, alcynylcarbonyle ou alcynyloxycarbonyle ayant chacun 2 à 6 atomes de carbone dans les groupes alcényle ou alcynyle et portant chacun, le cas échéant, un substituant fluoro, chloro ou bromo, un reste cyclo-alkyle ou cycloalkylalkyle ayant chacun 3 à 6 atomes de carbone dans les groupes cycloalkyle et, le cas échéant, 1 à 4 atomes de carbone dans la partie alkyle et portant chacun, le cas échéant, un substituant cyano, fluoro, chloro, bromo ou alkyle en C₁ à C₄,
R² représente l'hydrogène, un halogène, un groupe nitro, carboxy, cyano, thiocarbamoyle, amino, un reste alkyle, alkoxy, alkylthio, alkylamino ou dialkylamino ayant chacun 1 à 6 atomes de carbone dans les groupes alkyle et portant chacun, le cas échéant, un substituant cyano, fluoro, chloro, alkoxy en C₁ à C₄ ou alkylthio en C₁ à C₄, un reste alcényle, alcényloxy, alcénylthio, alcynyle, alcynyloxy ou alcynylthio ayant chacun 2 à 6 atomes de carbone dans les groupes alcényle ou alcynyle et portant chacun, le cas échéant, un substituant fluoro, chloro ou bromo, ou bien un reste cycloalkyle ou cycloalkylalkyle ayant chacun 3 à 6 atomes de carbone dans les groupes cyclo-alkyle et, le cas échéant, 1 à 6 atomes de carbone dans la partie alkyle et portant chacun, le cas échéant, un substituant cyano, fluoro, chloro, bromo ou alkyle en C₁ à C₄,
R³ représente le fluor, le chlore ou le brome,
R⁴ est un groupe cyano ou thiocarbamoyle et
R⁵ est le groupement -A¹-A²-A³,
dans lequel
A¹ représente l'oxygène,
A² est une liaison simple et
A³ est un reste alcényle ou alcynyle ayant chacun 2 à 6 atomes de carbone dans les groupes alcényle ou alcynyle et portant chacun, le cas échéant, un substituant fluoro ou chloro, ou bien
A¹ est le groupement -N-A⁴-,
A² est une liaison simple, et
A³ est un reste alkylsulfonyle ayant 1 à 6 atomes de carbone dans les groupes alkyle et portant, le cas échéant, un substituant fluoro, chloro ou alkoxy en C₁ à C₄, et
A⁴ est l'hydrogène ou un reste alkylsulfonyle en C₁ à C₄.

2. Procédé de production de 2-aryl-l,2,4-triazine-3,5-di(thi)ones de formule générale (I) 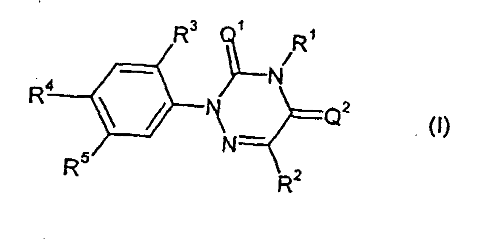 dans laquelle
R¹, R², R³, R⁴, R⁵, Q¹ et Q² ont les définitions mentionnées dans la revendication 1, **caractérisé en ce que**
(a) on fait réagir des 1,2,4-triazine-3,5-di(thi)ones de formule générale (II) 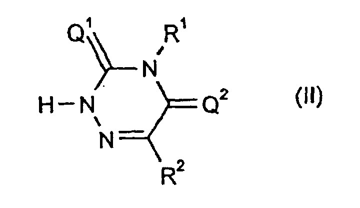 dans laquelle
Q¹, Q², R¹ et R² ont les définitions indiquées ci-dessus,
avec des dérivés halogénobenzéniques de formule générale (III) 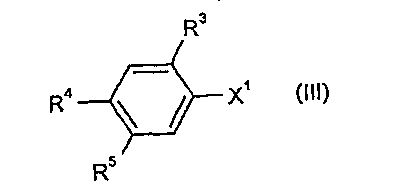 dans laquelle
R³, R⁴ et R⁵ ont les définitions indiquées ci-dessus et
X¹ est un halogène,
le cas échéant, en présence d'un diluant et en présence éventuelle d'un auxiliaire de réaction,
ou **en ce que**
(b) on fait réagir des 2-aryl-1,2,4-triazine-3,5-di(thi)ones de formule générale (Ia) 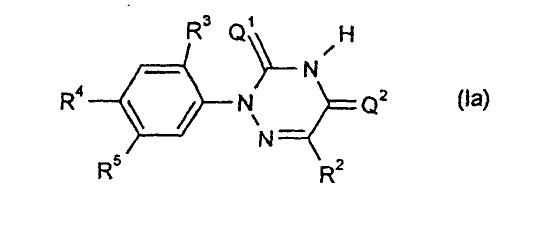 dans laquelle
Q¹, Q², R², R³, R⁴ et R⁵ ont les définitions indiquées ci-dessus,
avec des agents d'alkylation de formule générale (IV)
R¹-X² (IV)
dans laquelle
R¹ a la définition indiquée ci-dessus et
X² est un halogène ou le groupement O-SO₂-O-R¹,
le cas échéant, en présence d'un diluant et en présence éventuelle d'un auxiliaire de réaction,
ou **en ce que**
(c) on fait réagir des arylhydrazones de formule générale (V) 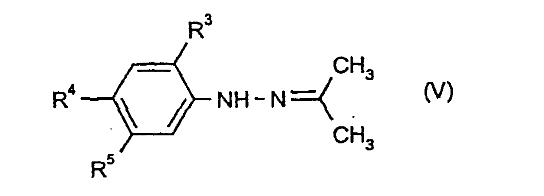 dans laquelle
R³, R⁴ et R⁵ ont les définitions indiquées ci-dessus,
avec des (thio)cyanates de métaux alcalins de formule générale (VI)
M-Q¹-CN (VI)
dans laquelle
Q¹ a la définition indiquée ci-dessus et
M est un atome de métal alcalin,
le cas échéant, en présence d'un diluant et en présence éventuelle d'un auxiliaire de réaction et on fait réagir les 2-aryl-1,2,4-triazolidine-3-(thi)ones ainsi formées, de formule générale (VII) 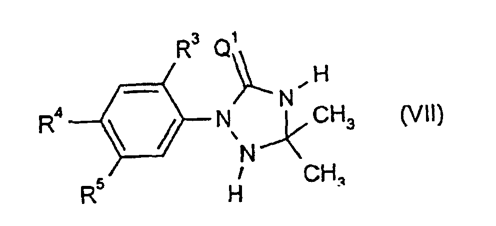 dans laquelle
Q¹, R³, R⁴ et R⁵ ont les définitions indiquées ci-dessus avec des acides cétocarboxyliques de formule générale (VIII) 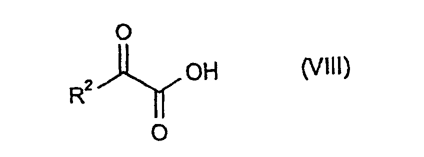 dans laquelle
R² a la définition indiquée ci-dessus,
le cas échéant, en présence d'un diluant et en présence éventuelle d'un auxiliaire de réaction,
ou **en ce que**
(d) on diazote des arylamines de formule générale (IX) 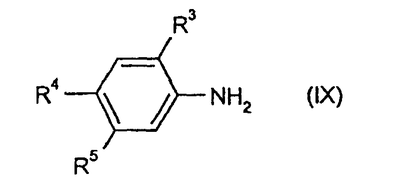 dans laquelle
R³, R⁴ et R⁵ ont les définitions indiquées ci-dessus
puis on conduit une réaction avec un ester d'acide cyanacétylcarbamique de formule générale (X) 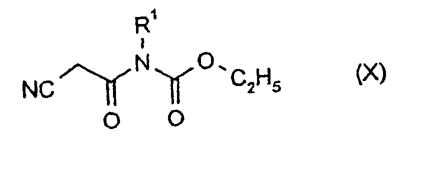 dans laquelle
R¹ a la définition indiquée ci-dessus,
le cas échéant, en présence d'un diluant et en présence éventuelle d'un auxiliaire de réaction,
et on cyclise les composés ainsi obtenus de formule générale (XI) 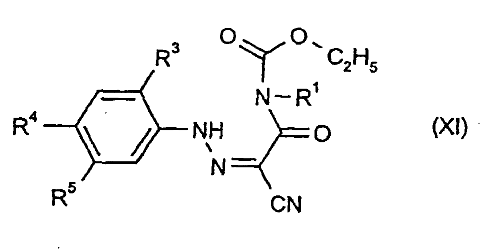 dans laquelle
R¹, R³, R⁴ et R⁵ ont les définitions indiquées ci-dessus
ou **en ce que**
(e) on fait réagir des arylhydrazines de formule (XII) 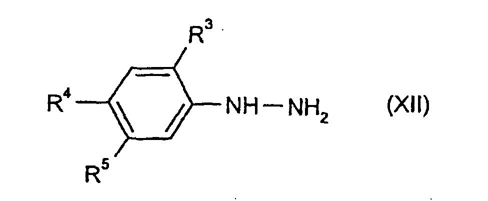 dans laquelle
R³, R⁴ et R⁵ ont les définitions indiquées ci-dessus,
avec des dérivés d'acides cétocarboxyliques de formule générale (XIII) 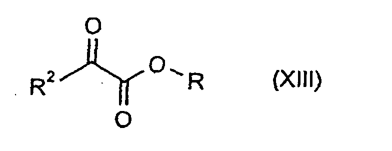 dans laquelle
R² a la définition indiquée ci-dessus et
R représente l'hydrogène ou un reste alkyle,
le cas échéant, en présence d'un diluant et en présence éventuelle d'un auxiliaire de réaction et on fait réagir les composés ainsi obtenus de formule générale (XIV) 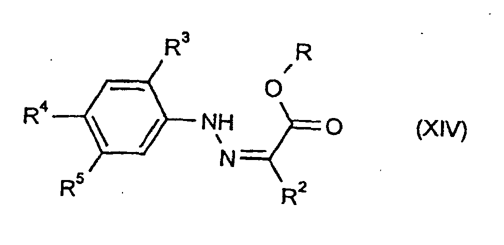 dans laquelle
R², R³, R⁴ et R⁵ ont les définitions indiquées ci-dessus et
R représente l'hydrogène ou un reste alkyle,
le cas échéant, en présence d'un diluant et en présence éventuelle d'un auxiliaire de réaction, avec des iso(thio)-cyanates de formule générale (XV)
R¹-N=C=Q¹ (XV)
dans laquelle
Q¹ et R¹ ont les définitions indiquées ci-dessus,
le cas échéant, en présence d'un diluant et en présence éventuelle d'un auxiliaire de réaction
et on cyclise les composés ainsi obtenus de formule générale (XVI) 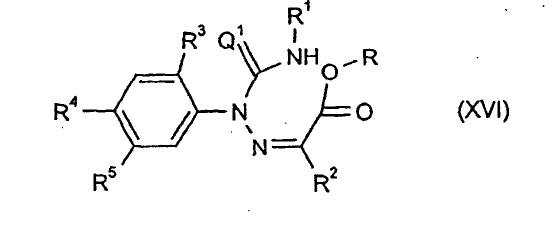 dans laquelle
Q¹, R¹, R², R³, R⁴ et R⁵ ont les définitions indiquées ci-dessus et
R représente l'hydrogène ou un reste alkyle,
le cas échéant, en présence d'un diluant et en présence éventuelle d'un auxiliaire de réaction, ou **en ce que**
(f) on obtient des acides 2-aryl-1,2,4-triazine-3,5-dione-6-carboxyliques de formule générale (XIX) 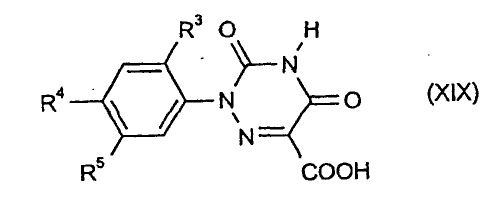 dans laquelle
R³, R⁴ et R⁵ ont la définition indiquée ci-dessus
lorsqu'on diazote des arylamines de formule générale (IX) 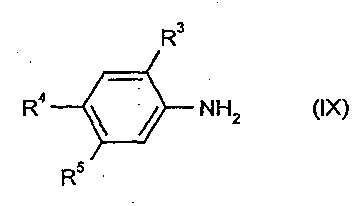 dans laquelle
R³, R⁴ et R⁵ ont les définitions indiquées ci-dessus,
puis on conduit une réaction avec le malonyldiuréthanne de formule (XVII)
CH₂(CO-NH-COOC₂H₅)₂ (XVII)
éventuellement en présence d'un diluant et, le cas échéant, en présence d'un auxiliaire de réaction, puis on conduit une cyclisation.

3. 2-aryl-l,2,4-triazine-3,5-di(thi)ones substituées de formule générale (I) suivant la revendication 1,
**caractérisées en ce que**
Q¹ représente l'oxygène ou le soufre,
Q² représente l'oxygène ou le soufre,
R¹ est l'hydrogène, un groupe cyano, amino, un reste méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylamino, éthylamino, n-propylamino, isopropylamino, n-butylamino, isobutylamino, sec.-butylamino, tertio-butylamino, diméthylamino, diéthylamino, acétyle, propionyle, n-butyroyle, isobutyroyle, méthoxycarbonyle, éthoxycarbonyle, n-propoxycarbonyle, isopropoxycarbonyle portant chacun, le cas échéant, un substituant cyano, fluoro, chloro, méthoxy ou éthoxy, un reste propényle, butényle, propénylcarbonyle, buténylcarbonyle, propényloxycarbonyle, butényloxy-carbonyle portant chacun, le cas échéant, un substituant fluoro, chloro ou bromo, un reste propynyle, butynyle, propynylcarbonyle, butynyl-carbonyle, propynyloxycarbonyle ou butynyloxy-carbonyle, ou un reste cyclopropyle, cyclobutyle, cyclopentyle, cyclohexyle, cyclopropylméthyle, cyclo-butylméthyle, cyclopentylméthyle ou cyclohexylméthyle portant chacun, le cas échéant, un substituant cyano, fluoro, chloro, bromo, méthyle ou éthyle,
R² représente l'hydrogène, un halogène, un groupe nitro, carboxy, cyano, thiocarbamoyle, amino, un reste méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, n-propylthio, isopropylthio, n-butylthio, isobutylthio, sec.-butylthio, tertio-butylthio, méthylamino, éthylamino, n-propylamino, isopropylamino, n-butylamino, isobutylamino, sec.-butylamino, tertio-butylamino, diméthylamino ou diéthylamino portant chacun, le cas échéant, un substituant cyano, fluoro, chloro, méthoxy ou éthoxy, un reste propényle, propényloxy, propénylthio, butényle, butényloxy ou buténylthio portant chacun, le cas échéant, un substituant fluoro, chloro ou bromo, un reste propynyle, propynyloxy, propynylthio, butynyle, butynyloxy ou butynylthio ou un reste cyclopropyle, cyclobutyle, cyclopentyle, cyclohexyle, cyclopropylméthyle, cyclobutylméthyle, cyclopentylméthyle ou cyclohexylméthyle portant chacun, le cas échéant, un substituant cyano, fluoro, chloro, bromo, méthyle ou éthyle,
R³ représente le fluor ou le chlore,
R⁴ est un groupe cyano ou thiocarbamoyle et
R⁵ représente le groupement -A¹-A²-A³,
dans lequel
A¹ est l'oxygène,
A² est une liaison simple et
A³ représente un reste propényle, butényle, propynyle ou butynyle dont chacun est éventuellement substitué par du fluor ou du chlore, ou bien
A¹ est le groupement -NH-,
A² est une liaison simple, et
A³ est un reste méthylsulfonyle, éthylsulfonyle, n-propylsulfonyle ou isopropylsulfonyle, chacun étant éventuellement substitué par du fluor, du chlore, un radical méthoxy ou éthoxy.

4. Compositions herbicides, **caractérisées par** une teneur en au moins une 2-aryl-1,2,4-triazine-3,5-di(thi)one substituée de formule générale (I) suivant la revendication 1 ou 3.

5. Procédé pour combattre une végétation indésirable, **caractérisé en ce qu'**on fait agir sur les plantes et/ou sur leur milieu des 2-aryl-1,2,4-triazine-3,5-di(thi)ones substituées de formule générale (I) suivant la revendication 1 ou 3.

6. Utilisation de 2-aryl-1,2,4-triazine-3,5-di(thi)ones substituées de formule générale (I) suivant la revendication 1 ou 3 pour combattre une végétation indésirable.

7. Procédé de préparation de compositions herbicides, **caractérisé en ce qu'**on mélange des 2-aryl-1,2,4-triazine-3,5-di(thi)ones substituées de formule générale (I) suivant la revendication 1 ou 3 avec des diluants et/ou des substances tensio-actives.

8. 2-aryl-1,2,4-triazine-3,5-di(thi)ones substituées de formule générale (I') 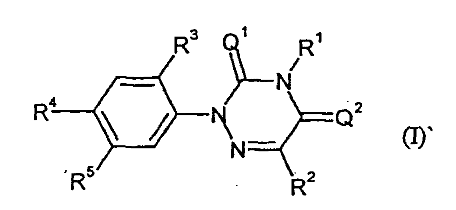 **caractérisées en ce que**
Q¹ représente l'oxygène ou le soufre,
Q² représente l'oxygène ou le soufre,
R¹ est l'hydrogène, un groupe cyano, amino, un reste alkyle, alkoxy, alkylamino, dialkylamino, alkyl-carbonyle ou alkoxycarbonyle ayant chacun 1 à 6 atomes de carbone dans les groupes alkyle, portant chacun, le cas échéant, un substituant cyano, fluoro, chloro, alkoxy en C₁ à C₄ ou alkylthio en C₁ à C₄, un reste alcényle, alcénylcarbonyle, alcényloxycarbonyle, alcynyle, alcynylcarbonyle ou alcynyloxycarbonyle ayant chacun 2 à 6 atomes de carbone dans les groupes alcényle ou alcynyle et portant chacun, le cas échéant, un substituant fluoro, chloro ou bromo, ou bien un reste cycloalkyle ou cycloalkylalkyle ayant chacun 3 à 6 atomes de carbone dans les groupes cycloalkyle et, le cas échéant, 1 à 4 atomes de carbone dans la partie alkyle et portant chacun, le cas échéant, un substituant cyano, fluoro, chloro, bromo ou alkyle en C₁ à C₄,
R² représente l'hydrogène, un halogène, un groupe nitro, carboxy, cyano, thiocarbamoyle, amino, un reste alkyle, alkoxy, alkylthio, alkylamino ou dialkylamino ayant chacun 1 à 6 atomes de carbone dans les groupes alkyle et portant chacun, le cas échéant, un substituant cyano, fluoro, chloro, alkoxy en C₁ à C₄ ou alkylthio en C₁ à C₄, un reste alcényle, alcényloxy, alcénylthio, alcynyle, alcynyloxy ou alcynylthio ayant chacun 2 à 6 atomes de carbone dans les groupes alcényle ou alcynyle et portant chacun, le cas échéant, un substituant fluoro, chloro ou bromo, ou bien un reste cycloalkyle ou cycloalkylalkyle ayant chacun 3 à 6 atomes de carbone dans les groupes cycloalkyle et, le cas échéant, 1 à 6 atomes de carbone dans la partie alkyle et portant chacun, le cas échéant, un substituant cyano, fluoro, chloro, bromo ou alkyle en C₁ à C₄,
R³ représente le fluor, le chlore ou le brome,
R⁴ est un groupe cyano ou thiocarbamoyle et
R⁵ est un groupe amino ou le fluor.

9. Composés de formule (I) suivant la revendication 1, dans lesquels les restes ont les définitions indiquées dans le tableau suivant :
| R¹ | R² | Q¹ | Q² | R³ | R⁴ | R⁵ |
|---|---|---|---|---|---|---|
| CH₃ | CH₃ | O | O | F | CN | OCH(CH₃)C≡CH |
| CH₃ | H | O | O | F | CN | OCH(CH₃)C≡CH |
| (CH₂)₂F | H | O | O | F | CN | OCH(CH₃)C≡CH |
| C₂H₅ | H | O | O | F | CN | N(C₂H₅)SO₂C₂H₅ |
| CH₃ | H | O | O | F | CN | N(SO₂C₂H₅)₂ |
| CH₃ | H | O | O | F | CN | NHSO₂C₂H₅ |
